# EUROPEAN PATENT SPECIFICATION

(11) **EP 0 651 759 B1**
(45) Date of publication and mention of the grant of the patent: **10.12.2003**
(21) Application number: 93917287.0
(22) Date of filing: 20.07.1993
(51) Int. Cl.: C07H 21/02, C07H 19/067, C07H 19/10, C07H 19/173, C07H 19/20, A61K 31/70, C07H 19/16

(54) **NOVEL 2'-O-ALKYL NUCLEOSIDES AND PHOSPHORAMIDITES PROCESSES FOR THE PREPARATION AND USES THEREOF**
2'-0-ALKYL-NUKLEOSIDE UND-PHOSPHORAMIDITE, VERFAHREN ZU IHRER HERSTELLUNG UND IHRE VERWENDUNGEN
NOUVEAUX 2'-O-ALKYLE NUCLEOSIDES ET PHOSPORAMIDITES, LEURS PROCEDES DE PREPARATION ET D'UTILISATION

(30) Priority: 23.07.1992 US 918362; 27.10.1992 US 967267; 30.10.1992 US 968849
(43) Date of publication of application: 10.05.1995
(60) Divisional application: 02076058.3
(73) Proprietor: ISIS PHARMACEUTICALS, INC., Carlsbad, CA 92008 (US)
(72) Inventor: McGEE, Daniel, Peter, Claude, Boulder, CO 80301 (US); COOK, Phillip, Dan, San Marcos, California 92069 (US); GUINOSSO, Charles, John, Vista, CA 92083 (US)
(74) Representative: Hallybone, Huw George
(86) International application number: US9306807
(87) International publication number: WO94002501

(56) References cited:
- DE-A- 4 110 085
- US-A- 5 214 135
- Proceedings of the National Academy of Sciences, Volume 87, issued October 1990, IRIBARREN et al., "2'-O-Alkyl Oligonucleotides as Antisense Probes", pages 7747-7751, especially page 7747, column 2, first sentence above "Materials and Methods".
- Nucleic Acids Research, Volume 19, No. 4, issued 1991, SPROAT et al., "New Synthetic Routes to Synthons Suitable for 2'-O-Allyl-Oligoribonucleotide Assembly", pages 733-738, esp. Abstract and page 733, column 1.
- Nucleic Acids Research, Vol. 19, No. 10, COTTEN et al., "2'-O-Methyl, 2'-O-Ethyl Oligonucleotides and Phosphorothioate Oligodeoxyribonucleotides as Inhibitors of the in Vitro UR snRNP-Dependent mRNA Processing Event", pages 2629-2635, esp. Abstract on page 2629.
- Nucleic Acids Research, Vol. 19, No. 21, issued 1991, WAGNER et al., "A Simple Procedure for the Preparation of Protected 2'-O-Methyl or 2'-O-Ethyl Ribonucleoside-3'-Phosphoramidites", pages 5965-5971, esp. page 5965, Abstract.
- Nucleic Acids Research, Vol. 18, No. 1, issued 1990, SPROAT et al., "New Synthetic Routes to Protected Purine 2'-O-Methylriboside-3'-O-Phosphoramidites Using a Novel Alkylation Procedure", pages 41-49, esp. page 41, columns 1-2.
- Nucleic Acids Research, Vol. 15, Number 15, 1987, INOUE et al., "Synthesis and Hybridization Studies on Two Complementary Nona(2'-O-Methyl)Ribonucleotides", pages 6131-6148, esp. page 6131, Abstract.
- Canadian Journal of Chemistry, Volume 59, issued 1981, ROBINS et al., "Nucleic Acid Related Compounds. 36. Synthesis of the 2'-O-Methyl and 3'-O-Methyl Ethers of Guanosine and 2'-Aminoadenosine and Correlation of O'-Methylnucleoside 13C nmr Specral Shifts", pages 3360-3364, see esp. the Reaction Scheme on page 3361.

## Description

### FIELD OF INVENTION

This invention is directed to processes for the preparation of 2'-*O*-substituted guanosines (in particular 2'-*O*-alkyl guanosines), 2'-*O*-alkyl guanosine 3'-*O*-phosphorarnidites and oligonucleotides that comprises at least one 2'-*O*-alkylated guanosine nucleotide.

### BACKGROUND OF THE INVENTION

A number of oligonucleotide analogs have been made. One class of oligonucleotides that have been synthesized are the 2'-*O*-substituted oligonucleotides. Such oligonucleotides have certain unique and useful properties. In United States patent application serial no. 814,961, filed December 24, 1991, entitled Gapped 2' Modified Phosphorothioate Oligonucleotides, assigned to the same assignee as this application, the entire contents of which are herein incorporated by reference, 2' substituted nucleotides are introduced within an oligonucleotide to induce increased binding of the oligonucleotide to a complementary target strand while allowing expression of RNase H activity to destroy the targeted strand.

In a recent article, Sproat, B.S., Beijer, B. and Iribarren, A., *Nucleic Acids Research*, **1990,** *18*:41, the authors noted further use of 2'-*O*-methyl substituted oligonucleotides as "valuable antisense probes for studying pre-mRNA splicing and the structure of spliceosomes".

2'-*O*-methyl and ethyl nucleotides and methods of making the same have been reported by a number of authors.

Robins, M.J., Naik, S.R. and Lee, A.S.K., *J. Org. Chem*., 39:1891 (1974) reported a low yield synthesis of 2'-*O*-and 3'-*O*-methyl guanosine via a stannous chloride catalyzed monomethylation by diazomethane. As was later reported by Robins, M.J., Hansske, F. and Bernier, S.E., *Can. J. Chem*., 59:3360 (1981), "convenient and high yield methods have been devised for synthesis of the 2'-*O*- and 3'-*O*-methyl ethers of adenosine, cytidine, and uridine... However, guanosine has presented significant difficulties." In the foregoing paper, the authors reported an improved synthesis of 2'-*O* and 3'-*O*-methyl guanosine. The synthesis was improved by effecting the stannous chloride catalyzed diazomethane methylation of 2,6-diamino-9-(β-*D*-ribofuranosyl)purine (2-aminoadenosine) in place of guanosine. The diamino purine moiety was then reduced to the corresponding guanine moiety with adenosine deaminase. In a further diazoation reaction described by Singer and Kusmierek, *Biochemistry* 15: 5052 (1976), a mixture of 2' and 3'-O-ethyl guanosine was reported to result from the treatment of guanosine with diazoethane. The alkylation also resulted in alkylation of the heterocyclic base . The alkylated product was treated with base to remove the ethyl group from the heterocyclic base. The resulting product was identified by virtue of having the same UV spectrum as that of guanosine, but a Rf differing from the Rf of guanosine.

A further improvement in the synthesis of 2'-*O*-methyl nucleosides was reported by Inoue, H., Hayase, Y. Imura, A., Iwai, S., Miura, K. and Ohtsuka, E., *Nucleic Acids Research*, 15:6131 (1987). This method of synthesis was effected utilizing CH₃I in the presence of Ag₂O. This method proved useful for all of the common nucleotides with the exception of guanosine. As reported by these authors, guanosine proved refractory to this synthetic method. Thus these authors again had to effect the 2'-*O*-methylation of guanosine with diazomethane. In order to avoid methylation of the amino functionality of the guanine base moiety, the guanine base moiety was blocked with an isobutyryl group. Additionally, to avoid methyl esterification of the 3'-*O* functionality of the sugar moiety, a TIPDS (tetraisopropyldisiloxane) blocking group was used to block both the 3' and the 5' hydroxyls of the sugar moiety.

Sproat et al., *supra* and Sproat, B.S., Iribarren, A.M., Garcia, R.G. and Beijer, B., *Nucleic Acids Research*, 19:733 (1991) addressed the synthesis of 2'-*O*-methyl guanosine (and 2'-*O*-allyl guanosine). In both of these Sproat et al. publications, the investigators presented a further synthetic pathway to 2'-*O*-methylguanosine and 2'-*O*-allylguanosine. They characterized the further pathway with respect to the prior known synthetic methods as "avoids(ing) ...the use of the highly toxic and potentially explosive reagent diazomethane" and being "far superior to the use of silver oxide/methyl iodide." This same synthetic method of the Sproat et al. investigators is also published in B.S. Sproat and A.I. Lamond, "2'-*O*-Methyloligoribonucleotides: synthesis and applications," *Oligonucleotides and Analogues*, ed. F. Eckstein, (IRL Press, 1991) which described syntheses of 2'-*O*-methylribonucleoside-3'-*O*-phosphoramidites. The uridine phosphoramidite synthesis described therein requires both base and sugar protection of the starting nucleoside prior to alkylation. Only after the base and sugar protecting groups are in place on the uridine is it then alkylated. Post alkylation, the base protecting group is removed followed by 5'-*O*-dimethoxytritylation and phosphitylation. The cytidine phosphoramidite synthesis described by Sproat and Lamond utilizes (and thus requires) the base and sugar blocked 2'-*O*-methyl uridine analog. This analog is then converted to a blocked cytidine analog, the blocking group is removed from the sugar, the analog is dimethoxytritylated and finally phosphitylated. The guanosine phosphoramidite synthesis taught by Sproat and Lamond starts from a 2-amino-6-chloronucleoside having 3' and 5' sugar hydroxy groups blocked. This nucleoside is converted to a 2,6-dichloro derivative. The dichloro compound is then 2'-*O* alkylated. Following *O*-alkylation, the dichloro compound is converted to a diazido intermediate. The diazido intermediate is in turn converted to a diamino intermediate. The diamino intermediate is then deaminated to the guanosine analogue. The 2-amino group of the guanosine analogue is blocked followed by dimethoxytritylation and finally phosphitylation. This guanosine procedure is also published in Sproat, et. al., *Nucleic Acids Research*, **1991** 19:733.

The above synthetic procedures involve multiple steps and numerous reagent treatments - 9 different reagent treatments for uridine, 10 for cytidine and 12 for guanosine. For the cytidine and guanosine compounds at least one of the reagents that is required is not readily available and thus is a very expensive reagent.

Other groups have taught the preparation of other 2'-O-alkylated nucleosides. 2'-*O*-methylthiomethylguanosine, was reported by Hansske, F., Madej, D. and Robins, M.J., *Tetrahedron*, 40:125 (1984). It was produced as a minor byproduct of an oxidization step during the conversion of guanosine to 9-β-*D*-arabinofuranosylguanine, i.e. the arabino analogue of guanosine. The addition of the 2'-*O*-methylthiomethyl moiety is an artifact from the DMSO solvent utilized during the oxidization procedure. The 2'-*O*-methylthiomethyl derivative of 2,6-diaminopurine riboside was also reported in the Hansske et al. publication. It was also obtained as an artifact from the DMSO solvent.

In addition, Gladkaya, et al., *Khim. Prir. Soedin*., **1989**, *4*, 568 discloses N₁-methyl-2'-*O*-(tetrahydropyran-2-yl) and 2'-O-methyl guanosine. Sproat, et al., *Nucleic Acids Research*, **1991**, *19*, 733 teaches the preparation of 2'-O-allylguanosine. Allylation of guanosine required a further synthetic pathway. Iribarren, et al., *Proc. Natl. Acad. Sci*., **1990**, *87*, 7747 also studied 2'-O-allyl oligoribonucleotides. Iribarren, et al. incorporated 2'-O-methyl-, 2'-O-allyl-, and 2'-O-dimethylallyl-substituted nucleotidesinto oligoribonucleotides to study the effect of these RNA analogues on antisense analysis. Iribarren found that 2'-O-allyl containing oligoribonucleotides are resistant to digestion by either RNA or DNA specific nucleases and slightly more resistant to nucleases with dual RNA/DNA specificity, than 2'-O-methyl oligoribonucleotides. However, Iribarren found that 2'-O-dimethylallyl containing oligoribonucleotides exhibited reduced hybridization to complementary RNA sequences as compared to 2'-O-methyl oligoribonucleotides. Thus, Iribarren suggested that further attempts to prepare alkylated RNA probes, especially those superior to 2'-allyl cytidine containing oligoribonucleotides should be limited to 2'-O-alkyl groups containing less than five carbon atoms.

Certain oligonucleotides containing 2'-*O*-alkyl substituted nucleotides are promising candidates for use as human pharmaceuticals. Those having long chain alkyl groups (i.e. four or more carbon atoms) are particularly useful. For example, long chain alkyl groups may accomodate functional groups in appropriate orientation with the opposing strand upon strand hybridization. Thus 2'-*O*- long chain alkyl nucleotides such as 2'-*O*- long chain alkyl guanosine nucleotides are highly desireable in some cases. For use in large scale therapeutic testing and eventually for human pharmaceutical use, large amounts of these oligonucleotides must be synthesized. The large amounts of oligonucleotides in turn requires large amounts of the 2'-*O*-alkyl nucleoside phosphoramidites used in synthesizing the oligonucleotides. Consideration must therefore be given to both cost and purity of the starting phosphoramidites used in the synthesis of such oligonucleotides. As a general premise, as the number of synthetic steps increases, the cost of manufacture increases. Further as the number of steps increases, quality control problems escalate. In view of this, it is evident that there is a great need for new and improved procedures for preparing nucleosides and nucleoside phosphoramidites.

### OBJECTS OF THE INVENTION

It is an object of this invention to provide methods of synthesis of 2'-*O*-alkylated nucleosides and nucleoside analogues.

It is an object of this invention to provide methods of synthesis of 2'-*O*- and 3'-*O*-alkylated 2,6-diaminopurine riboside compounds.

It is an object of this invention to provide new and improved synthetic methods for the preparation of 2'-*O*-alkyl nucleoside phosphoramidites.

It is an object of this invention to provide new and improved syntheses of 2'-*O*-alkyl guanosine phosphoramidites.

It is an object of this invention is to provide new and improved syntheses of 2,6-diamino-9-(2'-*O*-alkyl-β-*D*-ribofuranosyl)purine phosphoramidites.

It is an object of this invention to provide new and improved oligonucleotide syntheses that utilize the improved phosphoramidite syntheses of the invention.

These and other objects will become apparent to persons of ordinary skill in the art from a review of the present specification and appended claims.

### SUMMARY OF THE INVENTION

This invention includes processes for preparing compounds having the structure: 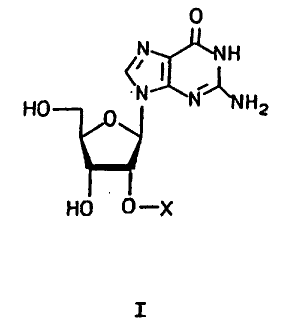 wherein X is R₁-(R₂)ₙ;
R₁ is C₃-C₂₀ alkyl, C₄-C₂₀ alkenyl or C₂-C₂₀ alkynyl;
R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; and n is an integer from 0 to about 6.

In other embodiments of the present invention, processes for preparing compounds having the structure : 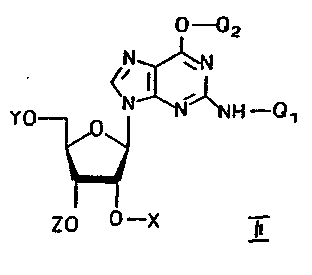 wherein X is R₁-(R₂)ₙ;
R₁ is C₃-C₂₀ alkyl;
R₂ is NH₂, H-imidazole, N-phthalimido;
Y is a hydroxyl blocking group;
Z is phosphate or an activated phosphate group;
Q₁ and Q₂ independently are H or a guanosine blocking group; and n is an integer from 0 to about 6, are also provided.

In still other embodiments of the present invention, processes are provided for preparing compounds having the structure: 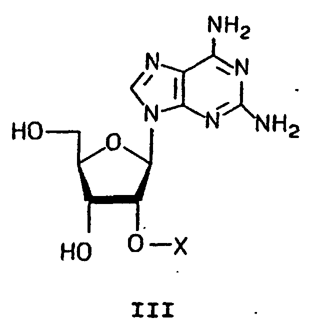 wherein X is R₁-(R₂)ₙ;
R₁ is C₃-C₂₀ alkyl, C₄-C₂₀ alkenyl or C₂-C₂₀ alkynyl;
R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, *O*-aralkyl, S-aralkyl, NH-aralkyl, amino, imidazole, N-phthalimido, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; and n is an integer from 0 to about 6.

This invention also includes processes for the facile preparation of 1'-*O*-monoalkyl or 2',3'-di-*O*-alkyl substituted guanosine compounds such as compounds of Formulas I, II and III. Except for preparation with diazomethane, heretofore, direct alkylation of guanosine has proven to be refractory. The present invention provides direct 2' and 3'-*O*-alkylation of 2,6-diamino-9-(-β-*D*-ribofuranosyl)purine, i.e. 2,6-diaminopurine riboside or 2-aminoadenosine, which can be effected followed by deamination of the 2'-*O*-alkylated 2,6-diamino purine riboside to the corresponding 2'-*O*-alkylated guanosine. This alkylation can be practiced, if desired, without the use of blocking groups on either the heterocycle or the sugar moieties of the nucleoside. Further unlike the use of diazomethane, which will only yield the methyl alkylation product, alkylation as practiced in this invention is not limited to just methyl alkylation but is used to yield a plenitude of alkyl substituted guanosine and 2,4-diaminopurine riboside compounds. The necessary compounds used in the invention having the formula R-L wherein R is an alkyl group and L is a leaving group, are either commercially available, are known in the literature or can be prepared by procedures analogous to known literature compounds.

The two step alkylation processes of the invention are further distinguished from the six step procedure of the Sproat et al. investigators. See the above-referenced *Nucleic Acids Research*, 18:41 (1990) and *Nucleic Acids Research*, 19:733 (1991) publications. In those procedures 2-amino-6-chloropurine riboside must first be blocked at both the 3' and 5' positions, converted to the 2,6-dichloro derivative, blocked at the 6 purine position, derivatized to the 2'-*O*-methyl or 2'-*O*-allyl derivative, converted to 2,6-diamino derivative, deblocked about the 3' and 5' positions and finally deaminated to the 2'-*O*-methyl or 2'-*O*-allyl guanosine product.

In accordance with the processes of this invention, alkylation is effected directly on 2,6-diamino-9-(β-*D*-ribofuranosyl)purine with an appropriate compound having the formula R-L, wherein R is an alkyl group and L is a leaving group, in the presence of a base of sufficient strength to effect removal of the proton from the 2' (or both 2' and 3') hydroxyl of the ribofuranosyl sugar moiety of 2,6-diamino-9- (β-*D*-ribofuranosyl)purine. Alkylation can be limited to mono alkylation by limiting the amount of either the R-L group or the base to a stoichiometric (or equivalent) amount. Alternately dialkylation (on both the 2' and 3' positions) can be practiced by use of an excess R-L group and base to concurrently alkylate both the 2' and the 3' positions.

While not wishing to be bound by theory, it has been observed that alkylation predominates at the 2' position compared to the 3' position. Generally a ratio of from about 7:3 to about 8:2 of 2' to 3' alkylation products are obtained (as determined by TLC). For both, TLC as well as preparative scale chromatography, the 2' product generally has a faster Rf than the 3' product. Advantage can be taken of this Rf difference to separate the 2'-*O*- and 3'-*O*- products from each other or from 2'-*O*-, 3'-*O*- dialkylated products. Thus the 2' and 3' alkylation products can be separated by procedures such as silica gel chromatography if desired.

For alkyl groups that are generally larger than propyl, further advantage can be taken of the rate of deamination of the 2' product versus the 3' product for separation of the 2'-*O* and 3'-*O* products. Thus mixtures of 2'-*O* and 3'-*O* alkylated 2,6-diamino-9-(β-*D*-ribofuranosyl)purine are subjected to deamination with adenosine deaminase. The enzymatic deamination of the 2'-*O* product is more facile than deamination of the 3'-*O* product. This difference in the rate of deamination allows for separation of the deaminated 2' product, i.e. the 2'-*O*-alkylated guanosine, from the slower or non-deaminated 3' product, i.e. the 2,6-diamino-9-(3'-*O*-alkylated-β-*D*-ribofuranosyl)purine. Additionally procedures such as crystallization have been utilized to further separate a 2' product from the corresponding 3' product by separating the 2'-*O*-alkylated diaminopurine riboside product from the corresponding 3'-*O*-alkylated diaminopurine riboside product.

A preferred base utilized for alkylation is sodium hydride. Other suitable bases may also be utilized, however such bases must have sufficient base strength to remove the proton from the 2' (and optionally 3') hydroxyl moiety of the 2,6-diaminopurine riboside starting material. While not wishing to be bound by theory, generally any base having a pKₐ about 10 pkₐ units greater than the pKₐ of the proton of the 2' hydroxyl moiety of the 2,6-diaminopurine riboside starting material may be used. More specifically, bases having a pK_{b} greater than the pK_{b} of sodium hydride may conveniently be selected. Such bases can be selected from compilations of base such as those given in Table 1, page 220 of March, J. *Advanced Organic Chemistry*, Wiley-Interscience, John Wiley & Sons, New York, 1985.

The alkylation reactions of the invention typically are conducted in DMF as the solvent. Other suitable solvents include DMSO, N-methyl pyrolidone and sulfolone.

Preferably, deamination is effected by use of deaminase enzymes. Particularly preferred is adenosine deaminase. Particularly suitable for use is Adenosine Deaminase Type II available from Sigma Chemical Company, St. Louis, MO. Other deamination reagents may also be employed. The deamination reactions of the invention typically are conducted in a mixture solvent containing an organic solvent and an aqueous buffer. Suitable for use as the organic solvent are DMSO, IN-methyl pyrolidone and sulfolone. In preferred embodiments of the present invention deamination is achieved using DMSO as the organic solvent. Suitable for use as the aqueous buffer are buffers having a pH compatible to the pH range of use of the deaminse enzyme. Preferred are phosphate buffers such as sodium phosphate and tris buffers.

In order to enrich the 2' product versus 3' product by elimination of any 3' product, a TIPDS (tetraisopropylsiloxane) protecting group is utilized to protect the 3' and 5' hydroxyl moieties of the sugar portions of the 2,6-diaminopurine riboside. In the same manner, exclusive 3' product would be obtainable by use of a base stable, non-migratory 2'-*O* protecting group. Such base stable, non-migratory protecting groups include but are not limited to tetrahydropyranyl (THP), 4-methoxytetrahydropyran-4-yl (Mthp), 1-[(2-chloro-4-methyl)phenyl-4-methoxypiperidin-4-yl (Ctmp), triphenylmethyl (trityl), mono-, di- and tri-methoxytrityl and other similar protecting groups.

In accordance with this invention there are also provided improved processes for the preparation of 2'-*O*-alkylated nucleoside phosphoramidites including 2'-*O*-alkylated guanosine phosphoramidites.

In accordance with methods of the present invention, preparation of a 2'-*O*-alkylated guanosine 3'*-O*-phosphoramidite may comprise the steps of alkylating a 2,6-diamino-9-(ribofuranosyl)purine to form a 2,6-diamino-9-(2'-*O*-alkylated ribofuranosyl)purine; deaminating said 2,6-diamino-9-(2'-*O*-alkylated ribofuranosyl)purine to form a 2'-*O*-alkylated guanosine; blocking the 5'-hydroxyl moiety of said 2'-*O*-alkylated guanosine; and phosphitylating the 3'-position of said 5'-blocked 2'-*O*-alkylated guanosine.

The 3'-*O*-phosphoramidite of 2'-*O*-alkyl guanosine and 2,6-diamino-9-(2'-*O*-alkyl-β-*D*-ribofuranosyl) purine can be provided in some embodiments of the present invention by reaction of 2-NH₂, 5'-OH protected 2'-*O*-alkyl guanosine or 2=NH₂, 6-NH₂, and 5'-OH protected 2,6-diamino-9-(2' -*O*-alkyl-β-*D*-ribofuranosyl) purine with commercially available reagent known to those skilled in the art, such as 2-cyanoethyl N,N-diisopropylaminochlorophosphine.

2'-O-alkyl guanosine and 2'-O-alkyl-2,6-diaminopurine riboside may be phosphitylated at the 3'-OH to provide phosphoramidites by methods known in the art, such as by protection of the NH₂ moieties (2- or 2- and 6- NH₂, respectively) and 5'-OH moiety, followed by reaction with cyanoethyl N,N-diisopropyl aminochlorophosphine.

Compounds prepared by a process of the present invention such as are provided herein can be incorporated into oligomers by procedures known to those skilled in the art.

In accordance with methods of the present invention an oligonucleotide that includes at least one 2'-*O*-alkylated guanosine nucleotide within the oligonucleotide may be prepared by processes comprising the steps of alkylating a 2,6-diamino-9-(ribofuranosyl)purine to form a 2,6-diamino-9-(2'-*O*-alkylated ribofuranosyl)purine; deaminating said 2,6-diamino-9-(2'-*O*-alkylated ribofuranosyl)purine to form a 2'-*O*-alkylated guanosine; blocking the 5'-hydroxyl moiety of said 2'-*O*-alkylated guanosine; phosphitylating the 3'-position of said 5'-blocked 2'-*O*-alkylated guanosine to form a 2'-*O*-alkylated guanosine 3'-*O*-phosphoramidite; and coupling, utilizing phosphoramidite coupling conditions, said 2'-*O*-alkylated guanosine 3'-*O*-phosphoramidite to a 5'-hydroxyl moiety of an oligonucleotide.

Oligomers prepared by a process of the present invention may contain at least one subunit having the structure: 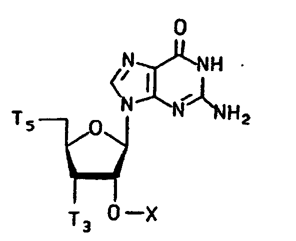 wherein X is R₁- (R₂)ₙ;
R₁ is C₃-C₂₀ alkyl, C₄-C₂₀ alkenyl C₂-C₂₀ alkynyl;
R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, imidazole, N-phthalimido, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether;
T₃ and T₅ independently are OH or a further nucleotide or nucleoside of said oligonucleotide or oligonucleoside that is joined to said structure; and
n is an integer from 0 to about 6.

In still other embodiments of the present invention, oligomers prepared by a process of the present invention may contain at least one subunit having the structure: 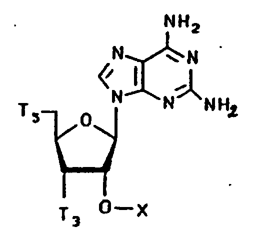 wherein X is R₁-(R₂)ₙ;
R₁ is C₁-C₂₀ alkyl, C₂-C₂₀ alkenyl C₂-C₂₀ alkynyl;
R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, imidazole, N-phthalimido, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether;
T₃ and T₅ independently are OH or a further nucleotide or nucleoside of said oligonucleotide or oligonucleoside that is joined to said structure; and
n is an integer from 0 to about 6.

Such oligomers or oligonucleotides may be prepared by solid state synthesis or by other means known to those skilled in the art.

In the context of this invention, the term "nucleoside" refers to a sugar and a base that are joined together, normally about an "anomeric" carbon on the sugar. Both α and β sugars are encompassed by the present invention. In preferred embodiments of the present invention the nucleoside sugar is a pentofuranosyl sugar, however, other sugars might also be utilized such as carbocyclic or 4'-deoxy-4'-thio sugar analogs.

In the context of this invention, the term "oligonucleotide" or "oligomer" refers to a polynucleotide formed from naturally occuring bases and furanosyl groups joined by native phosphodiester bonds. Oligonucleotides prepared by a process of the present invention will, of course, comprise at least one 2'-O-alkyl guanosine or derivative thereof. Thus, this term effectively refers to naturally occurring species or synthetic species formed from naturally occurring subunits or their close homologs. The term "oligonucleotide" or "oligomer" may also refer to moieties which have portions similar to naturally occurring oligonucleotides but which have non-naturally occurring portions. Thus, oligonucleotides may have altered sugars, altered base moieties, or altered inter-sugar linkages. Exemplary among these are the phosphorothioate and other sulfur-containing species which are known for use in the art. In accordance with some preferred embodiments, at least some of the phosphodiester bonds of the oligonucleotide have been substituted with a structure which functions to enhance the stability of the oligonucleotide or the ability of the oligonucleotide to penetrate into the region of cells where the messenger RNA is located. It is preferred that such substitutions comprise phosphorothioate bonds, phosphotriesters, methyl phosphonate bonds, short chain alkyl or cycloalkyl structures or short chain heteroatomic or heterocyclic structures. Other preferred substitutions are CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ structures where phosphodiester intersugar linkage is replaced by the substitutions. Also preferred are morpholino structures. Summerton, J.E. and Weller, D.D., U.S. 5,034,506 issued July 23, 1991. In other preferred embodiments, such as the protein-nucleic acid (PNA) backbone, the phosphodiester backbone of the oligonucleotide may be replaced with a polyamide backbone, the bases being bound directly or indirectly to the aza nitrogen atoms of the polyamide backbone. P.E. Nielsen, et al., *Science* **1991** *254* 1497. In accordance with other preferred embodiments, the phosphodiester bonds are substituted with other structures which are, at once, substantially non-ionic and non-chiral, or with structures which are chiral and enantiomerically specific. Persons of ordinary skill in the art will be able to select other linkages for use in practice of the invention.

Oligonucleotides may also include species which include at least some modified base forms. Thus, purines other than those normally found in nature may be so employed. Suitable bases include, but are not limited to those described in U.S. Patent 3,687,808. Similarly, modifications on the furanosyl portion of the nucleotide subunits, in addition to 2'-O-alkyl modifications of the present invention, may also be effected, as long as the essential tenets of this invention are adhered to. Examples of such modifications are 2'-halogen-substituted nucleotides. Some specific examples of modifications at the 2' position of sugar moieties which are useful in the present invention are OH, SH, SCH₃, F, OCN, O(CH₂)ₙNH₂, Cl, Br, CN, CF₃, OCF₃, S- or N- alkyl; S- or N-alkenyl; SOCH₃, SO₂CH₃; ONO₂; NO₂; N₃; NH₂; heterocycloalkyl; heterocycloalkaryl; aminoalkylamino; polyalkylamino; substituted silyl; an RNA cleaving group; a conjugate; a reporter group; an intercalator; a group for improving the pharmacokinetic properties of an oligonucleotide; or a group for improving the pharmacodynamic properties of an oligonucleotide and other substituents having similar properties. Sugar mimetics such as cyclobutyls may also be used in place of the pentofuranosyl group. Oligonucleotides may also comprise other modifications consistent with the spirit of this invention. Such oligonucleotides are best described as being functionally interchangeable yet structurally distinct from natural oligonucleotides. All such oligonucleotides are comprehended by this invention so long as they effectively function as subunits in the oligonucleotide.

Preferably oligonucleotides of the present invention are from about 6 to about 50 nucleotides in length. In still more preferred embodiments of the present invention oligonucleotides are from about 12 to about 20 nucleotides in length.

Further as used in this invention, the term "alkylating" refers to the addition of an alkyl, alkenyl or alkynyl moiety, preferably an alkyl moiety, to the precursors of the nucleosides phosphoramidites of the invention. Alkylation of the 2' position of the nucleoside sugar links the alkylating moiety to the 2' position of the sugar via an ether linkage.

Preferred alkyl moieties include un-substituted and substituted straight chain C₁-C₂₀ alkyl and un-substituted and substituted branch chain C₁-C₂₀ alkyl. Preferred alkenyl groups include un-substituted and substituted straight chain C₂-C₂₀ alkenyl, and un-substituted and substituted branch chain C₂-C₂₀ alkenyl. Preferred alkynyl groups include un-substituted and substituted straight chain C₂-C₂₀ alkynyl and un-substituted and substituted branch chain C₂-C₂₀ alkynyl. Thus preferred alkylation groups include but are not limited to C₁ to C₂₀ straight or branched chain lower alkyl or substituted lower alkyl, C₂ to C₂₀ straight or branched chain lower alkenyl or substituted lower alkynyl, C₂ to C₂₀ straight or branched chain lower alkynyl or substituted lower alkynyl.

Alkyl groups of the invention include, but are not limited to, C₁-C₂₀ straight and branched chained alkyls such as methyl, ethyl, propyl, butyl, pentyl, hexyl, heptyl, octyl, nonyl, decyl, undecyl, dodecyl, tridecyl, tetradecyl, pentadecyl, hexadecyl, octadecyl, nonadecyl and eicosyl, isopropyl, 2-butyl, isobutyl, 2-methylbutyl, isopentyl, 2-methyl-pentyl, 3-methylpentyl, 2-ethylhexyl and 2-propylpentyl. Alkenyl groups include, but are not limited to, unsaturated moieties derived from the above alkyl groups including, but not limited to, vinyl, allyl and crotyl. Alkynyl groups include unsaturated moieties having at least one triple bond that are derived from the above alkyl groups including, but not limited to, ethynyl and propargyl.

Substituent groups for the above include but are not necessarily limited to halogen (Cl, Br, F), hydroxyl (OH), thiol (SH), keto (C=O), carboxyl (COOH), nitrate (ONO₂), nitro (NO₂), nitroso (NO), nitrile (CN), trifluoromethyl (CF₃), trifluoromethoxy (OCF₃), O-alkyl, S-alkyl, NH-alkyl, N-dialkyl, O-aralkyl, S-aralkyl, NH-aralkyl, amino (NH₂), azido (N₃), hydrazino (NHNH₂), hydroxylamino (ONH₂), isocyanato (OCN), sulfoxide (SO), sulfone (SO₂), sulfide (S-), disulfide (S-S), silyl; heterocyclic, alicyclic, carbocyclic, intercalators, reporter molecules, conjugates, polyamines, polyamides, polyethylene glycols, and polyethers. Such compounds include 3-penten-2-one, 3-methyl-2-butanol, 2-cyanooctyl, 3-methoxy-4-heptanal, 3-nitrobutyl, 4-isopropoxydodecyl, 4-azido-2-nitrodecyl, 5-mercaptononyl, 4-amino-1-pentenyl as well as other substituted groups. These substituted groups can be introduced in a blocked or protected form and later de-blocked to the parent substituted compound. For example, use of the phthalimido group as a blocked form of an amino substitution is illustrated below.

Other suitable substituent groups include intercalators, reporter groups, reporter enzymes, and conjugates including cholesterols, phospholipids, biotin, phenanthroline, phenazine, phenanthridine, anthraquinone, acridones, pyrenes, stilbenes, oxazolo-pyridocarbazoles, anthraquinones, phenanthridines, phenazines, azidobenzenes, psoralens, porphyrins, cholic acids, folic acids fluoresceins, rhodamines, coumarins, and dyes; steroids, lipophilic molecules, peptides, protein, vitamins, RNA cleaving complexes, metal chelators, alkylators and cross-linking agents.

One particularly preferred substituent group is CF₃. Further particularly preferred substituent groups are phthalimido and imidazole. As noted, use of the phthalimido group allows for introduction of a blocked amino functionality on the alkyl group. Utilizing guanosine analogues prepared in accordance with this invention as intermediates in oligonucleotide synthesis, after oligonucleotide synthesis is complete, the phthalimido group is removed yielding an amino functionality tethered to a guanosine nucleotide within the oligonucleotide sequence. Use of an imidazole moiety as a substituent group on the alkyl functionality introduces the suggested nucleic acid cleaving functionality, imidazole, on a guanosine nucleotide within an oligonucleotide sequence.

Aryl groups include but are not limited to phenyl, tolyl, benzyl, naphthyl, anthracyl, phenanthryl, pyrenyl, and xylyl. Halogens include fluorine, chlorine and bromine. Suitable heterocyclic groups include, but are not limited to, imidazole, tetrazole, triazole, pyrrolidine, piperidine, piperazine and morpholine. Amines include amines of all of the above alkyl, alkenyl, alkynyl and aryl groups including primary and secondary amines and "masked amines" such as phthalimide. Amines are also meant to include polyalkylamino compounds and aminoalkylamines such as aminopropylamine and further heterocyclo-alkylamines such as imidazol-1, 2 or 4-yl-propylamine. RNA cleaving complexes may be, for example, intercalators or groups which bind in the minor groove of RNA. Intercalators are molecules which insert themselves between neighboring bases of an olignoucleotide. Reporter molecules are molecules which may aid in the identification of a molecule, either visually or otherwise. Cross-linking agents effectively join two groups.

Suitable leaving groups of the present invention include halides such as chloride, bromide, and iodide, sulfonates such as tosyl, brosyl, nosyl, mesyl and trifyl and oxonium ions. In preferred embodiments of the present invention the leaving group is a halide. Still other suitable leaving groups are well known to those skilled in the art.

In accordance with methods of the present invention, the alkylation is preferably conducted in the presence of a base, preferably a metal hydride such as sodium hydride. Additionally, the 5' hydroxyl blocking group is preferably a dimethoxytrityl moiety. The phosphitylating reagent is preferably bis-N,N-diisopropylaminocyanoethylphosphite and the phosphitylating reaction is preferably conducted in the presence of N,N-diisopropylamino-hydrotetrazolide.

In effecting the alkylation of guanosine, 2',6 diaminopurine is alkylated.

The amino moiety of the phosphoramidites of the invention can be selected from various amines presently used for such phosphoramidites. Such amines include both aliphatic and heteroaryl amines as are described in various United States patents, principally those to M. Caruthers and associates. These include United States patents 4,668,777, issued May 26, 1987; 4,458,066, issued Jul. 3, 1984; 4,415,732, issued Nov. 15, 1983; and 4,500,707, issued Feb. 19, 1985, all of which are herein incorporated by reference. One preferred amino group is diisopropylamino.

In addition to the amino moiety of the phosphoramidite, for phosphodiester and phosphorothioate linkages, an additional phosphorous blocking group is used. One preferred blocking group is the cyanoethyl group. Other phosphorous blocking groups include methoxy and 2-(methylsulphonyl)ethyl. Additionally an activating agent is normally used for the phosphoramidite coupling chemistry. One preferred activating agent is N,N-diisopropylaminohydrotetrazolide. Other suitable moieties for these functions are also disclosed in the above noted patents as well as in United States patent 4,725,677, issued Feb. 16, 1988 and Berner, S., Muhlegger, K., and Seliger, H., *Nucleic Acids Research* **1989**, *17*:853; Dahl, B.H., Nielsen, J. and Dahl, O., *Nucleic Acids Research* **1987**, *15*:1729; and Nielson, J. Marugg, J.E., Van Boom, J.H., Honnens, J., Taagaard, M. and Dahl, O., *J. Chem. Research* **1986**, 26, all of which are herein incorporated by reference.

For use in phosphorothioate linkage, the Beaucage reagent is described in Beaucage, S.L. and Caruthers, M.H., *Tetrahedron Letters* **1981**, *22*:1859 as well as in Zon, G. and Stec, J., Phosphorothioate oligonucleotides: *Oligonucleotides and Analogs A Practical Approach*; Eckstein, F. Ed.; IRL Press, Oxford, 1991, which also describes sulfurization by elemental sulfur.

Antisense therapy involves the use of oligonucleotides which are specifically hybridizable to target RNA or DNA. Oligonucleotides prepared by a process of the present invention are preferably specifically hydridizable with a target region. By "specifically hybridizable" herein is meant capable of forming a stable duplex with a target DNA or RNA. Upon binding to, or forming a stable duplex with, the target RNA or DNA, the antisense oligonucleotide can selectively inhibit the genetic expression of these nucleic acids or can induce some other events such as destruction of a targeted RNA or DNA or activation of gene expression. Destruction of targeted RNA can be effected by RNase H activation or by linking strand cleavers to the oligonucleotide. Antisense therapy is known in the art. See for example, PCT/US91/05720 filed December 3, 1991 entitled "Antisense Oligonucleotide Inhibitors of Papillomavirus" and PCT/US91/01327 filed February 25, 1991 entitled "Oligonucleotide Therapies for Modulating the Effects of Herpesvirus".

In some embodiments of the present invention the oligonucleotide portions of compounds prepared by a process of the present invention are at least 60% complementary to a target sequence. In preferred embodiments of the present invention, the oligonucleotide portions of compounds prepared by a process of the present invention are at least 80% complementary to a target sequence. 100% complementarity of the oligonucleotide portions of compounds prepared by a process of the present invention to a target sequence is most preferred. In preferred embodiments of the present invention, the oligonucleotide portions may be specifically hybridizable with DNA or RNA from *Candida*, papilloma virus, Epstein Barr virus, rhinovirus, hepatitis, human immunodeficiency virus, herpes simplex virus, influenza virus and cytomegalovirus.

2'-O-alkyl guanosine containing oligonucleotides prepared by a process of the present invention may be used to modulate the production of protein by contacting a selected sequence of RNA or DNA coding for a selected protein with an 2'-O-alkyl guanosine containing oligonucleotide having a sequence of nucleotide bases specifically hybridizable with said selected sequence of RNA or DNA coding for said protein.

The oligonucleotides prepared by a process of the present invention can be used in diagnostics, therapeutics and as research reagents. For therapeutic use, an animal having a disease characterized by the undesired production of a protein is contacted with an oligonucleotide prepared by a process of the present invention having a sequence of nucleotide bases specifically hybridizable with a selected sequence of RNA or DNA coding for said protein.

### EXAMPLES

The following examples illustrate the invention, however, they are not intended as being limiting. In various examples the nomenclature 4,4'-dimethoxytriphenylmethyl and dimethoxytrityl are used interchangeably to reference the DMT blocking group positioned on the 5'-hydroxyl moiety of the various nucleoside and nucleotides of the invention.

NMR spectra were obtained with the following instruments: ¹H-NMR: Varian Gemini-200 (199.975 MHz), ¹³C-NMR: Varian Gemini-200 (50.289 MHz). NMR spectra were recorded using either deuteriochloroform (tetramethylsilane as internal standard) or dimethylsulfoxide-*d*₆ as solvent. The following abbreviations were used to designate the multiplicity of individual signals: s = singlet, d = doublet, t =triplet, q = quartet, ABq = ab quartet, m = multiplet, dd = doublet of doublets, br s = broad singlet. Mass spectra were acquired on a VG 70-SEQ instrument (VG Analytical (Fisons)), using fast atom bombardment ionization (7 kV Xe atoms). Solvent ratios for column chromatography are given as volume/volume. Evaporations of solvents were performed *in vacuo* (60 torr) at 30°C unless otherwise specified. Melting points are reported uncorrected.

### EXAMPLE 1

### 2,6-Diamino-9-(β-D-ribofuranosyl)purine

In accordance with modifications of the procedures described in Robins, M.J., Hanske, F. and Beriner, S.E., *Can. J. Chem*., 59:3360 (1981), guanosine hydrate (49 g, Aldrich Chemical Co.), toluene (200 ml), hexamethyldisilazane (160 ml, 4.3 eq) and trifluoromethanesulfonic acid (3.7 ml) were loaded in a stainless steel Parr bomb. The bomb was sealed and heated approximately 1/3 submerged in an oil bath at 170° C for 5 days. The bomb was cooled in a dry ice acetone bath and opened. The contents were transferred to a 2 liter round bottom flask using methanol (MeOH) and the solvent evaporated on a Buchii evaporator. 1:1 H₂O/MeOH (600 ml) was added to the residue and the resulting brown suspension was refluxed 4-5 hr. The resulting suspension was evaporated on the Buchii evaporator to remove the methanol (≈ 1/2 volume). Additional H₂O (≈300 ml) was added and the mixture was heated, treated with charcoal and filtered through a Celite filter pad. Upon cooling, a crystalline solid formed. The solid was isolated by filtration, washed with H₂O and dried under high vacuum at 90° C to yield the product (43.7 g, 89% yield) as a tan solid. UV and NMR spectra of this compound compared to literature values.

This variation of the procedures of Robins, et al. supra, eliminated the need to utilize liquid ammonia in the reaction mixture since the ammonia molecule is generated *in situ* from the silazane reagent and the water of hydration of the guanosine hydrate starting material. Further, the use of chlorotrimethylsilane was not necessary nor was it necessary to conduct the reaction under anhydrous conditions, do a preliminary evaporation, or open and re-seal the Parr bomb under a dry nitrogen atmosphere.

### EXAMPLE 2

### 2,6-Diamino-9-(2'-O-propyl-β-D-ribofuranosyl)purine & 2,6-Diamino-9-(3'-O-propyl-β-D-ribofuranosyl)purine

Sodium hydride (NaH) (2.1 g) was added to 2,6-diamino-9-(β-*D*-ribofuranosyl) purine (10.5 g) in dry dimethylformamide (DMF) (150 ml). After stirring for 10 min, iodopropane (6 ml) was added. The solution was stirred for 45 min at room temperature followed by the addition of a further aliquot of NaH (600 mg). The reaction mixture was stirred overnight and then quenched by the addition of ethanol (EtOH) (5 ml). The reaction mixture was evaporated in vacuo, the residue suspended in 10% MeOH/CH₂Cl₂ and purified by silica gel chromatography (300 g) using 5 → 10% MeOH/CH₂Cl₂ as the eluent . The 2',3'-di-*O*-propyl product eluted first followed by the 2'-*O*-propyl product and then the 3'-*O*-propyl product. The 2'-*O*-propyl product containing fractions were pooled and the solvent stripped to yield a crude foam. The foam was crystallized from H₂O (40 ml), washed with cold H₂O and dried to yield 2.9 g of the 2'-*O*-propyl compound. The mother liquor was evaporated, re-chromatographed and crystallized to yield an additional 2.4 g of the 2'-*O*-propyl compound. The second mother liquor was evaporated to yield 4 g of a mixture of 2' and 3'-*O*-propyl compounds as an oil. Fractions containing the 3'-*O*-propyl product as the major product were evaporated and residue crystallized from water. (See Example 17 below for isolation and characterization of the 2',3'-di-*O*-propyl compound).

### 2,6-Diamino-9-(2'-O-propyl-β-D-ribofuranosyl)purine

¹H NMR (DMSO-d₆) δ 0.76 (t, 3, CH₃), 1.4 (tq, 2, CH₂), 3.3 (m, 1, H-5'' + HDO), 3.65-3.45 (m, 3, H-5', O-CH₂), 3.9 (m, 1), 4.25 (br m, 1), 4.38 (dd, 1), 5.1 (br d, 1 3'-OH), 5.45 (br t, 1, 5'-OH), 5.75 (br s, 2, 6-NH₂), 5.83 (d, 1, H-1'), 6.77 (br s, 2, 2-NH₂) and 7.95 (s, 1, H-8). Anal. Calcd. for C₁₃H₂₀N₆O₄·½H₂O: C, 46.91; H, 6.2; N,25.25. Found: C, 47.09; H, 6.37; N, 25.33.

### 2,6-Diamino-9-(3'-O-propyl-β-D-ribofuranosyl)purine

¹H NMR (DMSO-d₆) δ 0.75 (t, 3, CH₃), 1.4 (tq, 2, CH₂), 3.27-3.5 (ABX 2, O-CH₂-), 3.5 and 3.6 (ABX, 2, H-5'), 3.9 (m,1), 4 .22 (m, 1) , 4.35 (m, 1) , 5.1 (br d, 1, 2' -OH) , 5.45 (br t, 1, 5'-OH), 5.75 (br s, 2, 6-NH₂), 5.8 (d, 1, H-1'), 6.8 (br s, 2CH₂, 2-H₂) and 7.95 (s, 1, H-8).

### EXAMPLE 3

### 2'-O-Propylguanosine

A mixture of 2,6-Diamino-9-(2'-*O*-propyl-β-*D*-ribofuranosyl) purine and 2,6-Diamino-9-(3'-*O*-propyl-β-*D*-ribofuranosyl) purine (4.6 gm) and adenosine deaminase (200 mg, Sigma Chemicals Type II) were stirred at room temperature overnight in 0.1 M tris buffer (150 ml, pH 7.4), DMSO (100 ml) and 0.1 M sodium phosphate buffer (10 ml). A further aliquot of adenosine deaminase (140 mg) in 0.1 M phosphate buffer (30 ml) and DMSO (20 ml) was added and the reaction stirred an addition 24 hrs. The solvent was evaporated in vacuo and the residue flash chromatographed on silica gel utilizing 5 → 20% MeOH/CH₂Cl₂. Product-containing fractions were evaporated in vacuo and the residue crystallized from H₂O to yield 2.6 gm of product. m.p. dec > 270° C. ¹H NMR (DMSO-d₆) δ 0.75 (t, 3, CH₃), 1.42 (tq, 2, CH₂), 3.3-3.6 (m, 4, H-5', O-CH₂), 3,85 (m, 1), 4. 2 (m, 1), 4.23 (m, 1), 5.10 (t, 1, 5'-OH), 5.13 (d, 1, 3'-OH), 5.75 (d, 1, H-1'), 6.45 (br s, 2, NH₂), 7.95 (s, 1, H-8) and 10 . 67 (br s, 1, NH). Anal. Calcd. for C₁₃H₁₉N₅O₅: C, 47.99; H, 5.89; N, 21.53. Found: C, 47.90, H, 5.85; N, 21.44.

### EXAMPLE 4

### N2-Isobutyryl-2'-O-propylguanosine

2'-*O*-Propylguanosine (3.6 gm) in pyridine (50 ml) was cooled in an ice bath and trimethylsilyl chloride (8.4 ml, 6 eq.) was added. The reaction mixture was stirred for 30 min and isobutyryl chloride (5.8 ml, 5 eq.) was added. The solution was stirred for 4 hours during which it was allowed to warm to room temperature. The solution was cooled, H₂O added (10 ml) and the solution was stirred for an additional 30 mins. Concentrated NH₄OH (10 ml) was added and the solution evaporated in vacuo. The residue was purified by silica gel chromatography using 10% MeOH/CH₂Cl₂ to elute the product. Product-containing fractions were evaporated to yield 2.5 g of product as a foam. An analytical sample was re-chromatographed on silica and eluted with CH₂Cl₂ → 6% MeOH/CH₂Cl₂. ¹H NMR (DMSO-d₆) δ 0.75 (t, 3, CH₃), 1.13 [d, 6, CH(CH₃)₂], 1.4 (m, 2, CH₂), 2.75 [m, 1, CH(CH₃)₂], 3.52 (m, 6, OCH₂), 3 .36 and 3.6 (ABX, 2, H-5'), 3 .95 (m, 1), 4 .26 (m, 1), 4.33 (m, 1), 5.07 (t, 1, 5'-OH), 5.18 (d, 1, 3'-OH), 5.9 (d, 1, H-1'), 8.25 (s, 1, H-8), 11.65 (br s, 1, NH) and 12.1 (br s, 1, NH). Anal. Calcd. for C₁₇H₂₅N₅O₆·½H₂O: C, 50.49; H, 6.48; N, 17.32. Found: C, 50.81; H, 6.62; N, 17.04.

### EXAMPLE 5

### N2-Isobutyryl-5'-dimethoxytrityl-2'-O-propylguanosine

N2-Isobutyryl-2'-*O*-propylguanosine (2.64 g) was co-evaporated with pyridine and then solubilized in pyridine (180 ml). Dimethoxytrityl chloride (2.4 g, 1.1 eq) and dimethylaminopyridine (50mg) were added with stirring at room temperature. The reaction mixture was stirred overnight and evaporated in vacuo. The residue was partitioned between CH₂Cl₂/ 2x dil Na₂CO₃. The organic phase was dried (MgSO₄) and evaporated. The residue was purified by silica gel chromatography (1:1 EtOAc/Hex → 5% MeOH/EtOAc, 1% TEA) to yield 4.1 g of product. ¹H NMR (DMSO-d₆) δ 0.78 (t, 3, CH₃), 1.12 [d, 6, CH(CH₃)₂], 1.46 (m, 2, CH₂), 2.75 [m, 1, CH(CH₃)₂], 3 .35 and 3.55 (ABX, 2, H-5'), 3.73 (s, 6, OCH₂), 4.0 (m, 1), 4.3 (m, 1), 4.4 (m, 1), 5.18 (d, 1, 3'-OH), 5.93 (d, 1, H-1'), 6. 8, 7. 2, 7.36 (m, 13, DMTr) , 8.13 (s, 1, H-8), 11.63 (br s, 1, NH) and 12.1 (br s, 1, NH). Anal. Calcd. for C₃₈H₄₂N₅O₈·H₂O: C, 63.83; H, 6.20; N, 9.80. Found: C, 64.22; H, 6.35; N, 9.55.

### EXAMPLE 6

### N2-Isobutyryl-5'-dimethoxytrityl-2'-O-propylguanosine 3'-β-cyanoethyl-N,N-diisopropylphosphoramidite

A CH₂Cl₂ solution of N2-Isobutyryl-5'-dimethoxytrityl-2'-*O*-propylguanosine (4.1 g), *bis*-(N,N-diisopropylamino)-2-cyanoethylphosphite (3.7 ml, 2 eq) and N,N-diisopropylammonium tetrazolide (0.5 g, 0.5 eq) was stirred at room temperature overnight . The solution was partitioned against dil. Na₂CO₃ and then dil. Na₂CO₃/NaCl and dried over MgSO₄. The solvent was evaporated in vacuo and the residue was purified by silica gel chromatography (120 g, 1%TEA in EtOAc) to yield 5.2 g of product as a foam. ³¹P NMR (CDCl₃) δ 150.5, 150.8.

### EXAMPLE 7

### 2,6-Diamino-9-(2'-O-pentyl-β-D-ribofuranosyl)purine & 2,6-Diamino-9-(3'-O-pentyl-β-D-ribofuranosyl)purine

2,6-Diamino-9-(β-*D*-ribofuranosyl)purine (10 g) was treated with sodium hydride (1.7 g, 1.2 eq) and bromopentane (5.3 ml, 1.2 eq) in DMF (90 ml) as per the procedure of Example 2. Silica gel chromatography yielded three components. The first eluted component (not characterized but believed to be the 2,3-di-(*O*-pentyl) compound was isolated as an oil (700 mg). The next component isolated as a foam (3.3 g) was crystallized from MeOH to yield 2.8 g of 2,6-diamino-9-(2'-*O*-pentyl-β-*D*-ribofuranosyl)purine. The third component isolated as a solid (200 mg) was crystallized from MeOH to yield 80 mg of 2,6-diamino-9-(3'-*O*-pentyl-β-*D*-ribofuranosyl)purine. Fractions containing mixtures of the first and second components were evaporated and the residue crystallized from MeOH to yield a further 900 mg of the 2-*O*-pentyl compound. Further fraction yielded 1.2 g of a mixture of the 2'-*O*-pentyl and 3'-*O*-pentyl compounds.

### 2,6-Diamino-9-(2'-O-pentyl-β-D-ribofuranosyl)purine

¹H NMR (DMSO-d₆) δ 0.75 (t, 3, CH₃), 1.16 (m, 4, CH₂), 1.39 (m, 2, CH₂), 3.53 (m, 2, CH₂), 3.3 and 3.6 (ABX, 2, H-5'), 3.93 (br s, 1), 4.23 (m, 1), 4.38 (m, 1), 5.1 (d, 1 3'-OH), 5.5 (t, 1, 5'-OH), 5.75 (br s, 2, 6-NH₂), 5.82 (d, 1, H-1'), 6.8 (br s, 2, 2-NH₂) and 7.93 (s, 1, H-8).

### 2,6-Diamino-9-(3'-O-pentyl-β-D-ribofuranosyl)purine

¹H NMR (DMSO-d₆) δ 0.87 (t, 3, CH₃), 1.3 (m, 4, CH₂), 1.55 (m, 2, CH₂), 3.5 (m, 2, O-CH₂-), 3.6 (m, 2, H-5'), 3.86 (m, 1), 3.95 (m, 1) , 4. 6 (m, 1) , 5.32 (br d, 1 2'-OH), 5.46 (br t, 1, 5'-OH), 5.70 (d, 1, H-1'), 5.75 (br s, 2, 6-NH₂), 6.76 (br s, 2, 2-NH₂) and 7.93 (s, 1, H-8).

### EXAMPLE 8

### 2'-O-Pentylguanosine

2,6-diamino-9-(2'-*O*-pentyl-β-*D*-ribofuranosyl)purine (1.9 g) in 0.1 M sodium phosphate buffer (50 ml, pH 6.0) and DMSO (25 ml) was treated with adenosine deaminase (added in two aliquots - first aliquot 50 mg, second aliquot 80 mg) at 35° C as per the procedure of Example 3 to yield 1.4 g of product. ¹H NMR (DMSO-d₆) δ 0.8 (t, 3, CH₃), 1.16 (m, 4, 2x CH₂), 1.4 (m, 2, CH₂), 3 .38, 3.6 (m, 4, OCH₂, H-5'), 3.93 (s, 1, H-4'), 4.28 (m, 2, H-2', H-3'), 5.17 (br, 2, 5', 3'-OH), 5.8 (d, 1, H-1'), 6.53 (br s, 2, NH₂), 8.0 (s, 1, H-8) and 10.68 (br, 1, NH).

### EXAMPLE 9

### N2-Isobutyryl-2'-O-pentylguanosine

2'-*O*-pentylguanosine (2.3 g) in pyridine (35 ml) was treated with trimethylsilyl chloride (4.15 ml, 5 eq) and isobutyryl chloride (3.4 ml, 5 eq) as per the procedure of Example 4 to yield the product as a foam (2.3 g). An analytical sample was crystallized from EtOAc/Hex. m.p. 178-180° C. ¹H NMR (DMSO-d₆) δ 0.75 (t, 3, CH₃), 1.1 [m, 10, 2x CH₂, CH(CH₃)₂], 1.4 (m, 2, CH₂), 2.74 [m, 1, CH(CH₃)₂], 3.56 (m, 4, OCH₂, H-5'), 3.93 (m, 1, H-4'), 4.25 (m, 1), 4.34 (m, 1), 5.05 (t, 1, 5'-OH), 5.17 (d, 1, 3'-OH), 5.88 (d, 1, H-1'), 8.27 (s, 1, H-8), 11.65 (br s, 1, NH) and 12.05 (br s, 1, NH) . Anal. Calcd. for C₁₉H₂₉N₅O₆: C, 53.89; H, 6.90; N, 16.54. Found: 53.75; H, 6.92; N, 16.40

### EXAMPLE 10

### N2-Isobutyryl-5'-dimethoxytrityl-2'-O-pentylguanosine

N2-Isobutyryl-2'-*O*-pentylguanosine (2.3 g) was treated with dimethoxytrityl chloride (1.7 g, 1.1 eq), and dimethylaminopyridine (100 mg as a catalyst) in pyridine (50 ml) as per the procedure of Example 5 to yield the product as a foam (2.9 g). ¹H NMR (DMSO-d₆) δ 0.83 (t, 3, CH₃), 1.2 [m, 10, 2x CH₂, CH(CH₃)₂], 1.48 (m, 2, CH₂), 2.78 [m, 1, CH(CH₃)₂], 3.4, 3.6 (m, 4, OCH₂, H-5'), 3.75 (s, 6, OCH₃), 4.07 (m, 1), 4.27 (m, 1), 4.42 (m, 1), 5.2 (br d, 1, 3'-OH), 5.95 (d, 1, H-1'), 6.85, 7.25, 7.38 (m, 13, DMTr), 8.15 (s, 1, H-8), 11.67 (br s, 1, NH) and 12.1 (br s, 1, NH). Anal. Calcd. for Anal. Calcd. for C₄₀H₄₇N₅O₈·½H₂O: C, 65.38; H, 6.58; N, 9.53. Found: C, 65.37; H, 6.59; N, 9.39.

### EXAMPLE 11

### N2-Isobutyryl-5'-dimethoxytrityl-2'-O-pentylguanosine 3'-β-cyanoethyl-N,N-diisopropylphosphoramidite

N2-Isobutyryl-5'-dimethoxytrityl-2'-*O*-pentyl-guanosine (1.7 g) was treated with *bis*-(N,N-diisopropylamino)-2-cyanoethyl-phosphite (1.48 g) and N,N-diisopropylammonium tetrazolide (200 mg) as per the procedure of Example 6 to yield the product (1.4 g). ³¹P NMR (CDCl₃) δ 150.5, 150.85.

### EXAMPLE 12

### 2,6-Diamino-9-(2'-O-nonyl-β-D-ribofuranosyl)purine

2,6-Diamino-9-(β-*D*-ribofuranosyl)purine (50 g, 180 mmol) was treated with sodium hydride (8.8 g, 220 mmol) and bromononane (59 g, 54.4 ml, 285 mmol) in DMF (700 ml) as per the procedure of Example 2 (the diamino compound in DMF was cooled in an ice bath during the addition of NaH) to yield 83 g of crude product. 50 g of crude product was purified by silica gel chromatography. Fraction containing 2'-*O*-nonyl and 3'-*O*-nonyl product were combined to give a 77:23 mixture (29 g) of the 2' and 3' product. Pure 2'-*O*-nonyl product is obtained by chromatography. ¹H NMR (DMSO-d₆) δ 0.95 (t, 3, CH₃); 1.17 [m, 12, O-CH₂-CH₂-(CH₂)₆], 1.42 [m, 2, O-CH₂CH₂(CH₂)₆]; 3.27-3.70 (m, 2, H-5'); 3.50-3.70 [m, 2, O-CH₂(CH₂)₇]; 3.95 (m, 1, H-4'), 4.24 (m, 1, H-3'); 4.40 (m, 1, H-2'); 5.10 (d, 1, 3'-OH, *J*= 5 Hz); 5.50 (t, 1, 5'-OH, *J*= 6 Hz); 5.76 (s, 2, 2-NH₂); 5.83 (d, 1, H-1', *J*= 6.0 Hz); 6.81 (s, 2, 6-NH₂); and 7.96 (s, 1, 8-H).

### EXAMPLE 13

### 2'-O-Nonylguanosine

A mixture of 2,6-diamino-9-(2'-*O*-nonyl-β-*D*-ribofuranosyl)purine and 2,6-diamino-9-(3'-*O*-nonyl-β-*D*-ribofuranosyl)purine (≈ 80:20 mixture, 29 g) in 0.1 M sodium phosphate buffer (50 ml, pH 7.4), 0.1 M tris buffer (1800 ml, pH 7.4) and DMSO (1080 ml) was treated with adenosine deaminase (1.6 g) as per the procedure of Example 3 to yield 60 g of product as an oil. An analytical product was purified by silica gel chromatography and recrystallized from EtOAc. m.p. 258-259° C. ¹H NMR (DMSO-d₆) 6 0.96 (t, 3, CH₃, *J*= 7 Hz); 1.17 [m, 12, O-CH₂-CH₂-(CH₂)₆]; 1.42 [m, 2, O-CH₂CH₂(CH₂)₆]; 3.27-3.61 (m, 4, H-5', O-CH₂(CH₂)₇]; 3.95 (m, 1, H-4'), 4.10-4.13 (m, 2, H-2', H-3'); 5.13-6. 06 (m, 2, 3'-OH 5'-OH); 5.80 (d, 1, H-1', *J*= 6.4 Hz); 6.47 (s, 2, 2-NH₂); 7.98 (s, 1, 8-H) and 10.64 (s, 1, N₁ amide). Anal. Calcd. for C₁₉H₃₁N₅O₅: C, 55.73; H, 7.63; N, 17.10. Found: C, 55.67; H, 7.66; N, 17.02.

### EXAMPLE 14

### N2-Isobutyryl-2'-O-nonylguanosine

2'-*O*-nonylguanosine (14.7 g) in pyridine (360 ml) was treated with trimethylsilyl chloride (23.4 ml) and isobutyryl chloride (30.6 ml) as per the procedure of Example 4 to yield crude product (37 g). The crude material was purified by silica gel chromatography (eluted with 90/10 CHCl₃/MeOH) to yield 14.6 g of product re-crystallized from EtOAc. m.p. 168-169° C. ¹H NMR (DMSO-d₆) δ 0.85 [t, 3, CH₃(nonyl)], 1.14 [m, 18, O-CH₂CH₂(CH₂)₆, CH(CH₃)₂], 1.40 [m, 2, O-CH₂CH₂(CH₂)₆], 2.79 [m, 1, CH(CH₃)₂], 3.31-3.63 (m, 4, H-5', O-CH₂(CH₂)₇]; 3.96 (m, 1, H-4'), 4.27-4.37 (m, 2, H-2', H-3'); 5.10 (t, 1, 5'-OH, *J*= 5 Hz), 5.18 (d, 1, 3'-OH, *J*= 4 Hz), 5.91 (d, 1, H-1', *J*= 6.6 Hz), 8.31 (s, 1, 8-H), 11.73 (s, 1, C₂ amide) and 12.11 (s, 1, N₁ amide). Anal. Calcd. for C₂₃H₃₇N₅O₆: C, 57.60; H, 7.78; N, 14.60. Found: C, 57.63; H, 7.92; N, 14.62.

### EXAMPLE 15

### N2-Isobutyryl-5'-dimethoxytrityl-2'-O-nonylguanosine

N2-Isobutyryl-2'-*O*-nonylguanosine (14.6 g, 30.4 mmol) was treated with dimethoxytrityl chloride (12.1 g, 34 mmol) in pyridine (200 ml) as per the procedure of Example 5 to yield 16 g of purple foam prior to chromatography and 11.5 g after chromatography purification. ¹H NMR (DMSO-d₆) δ 0.84 [t, 3, CH₃(nonyl), *J*= 7 Hz], 1.16 [m, 18, O-CH₂CH₂(CH₂)₆, CH(CH₃)₂], 1.43 [m, 2 , O-CH₂CH₂(CH₂)₆], 2.77 [m, 1, CH(CH₃)₂], 3.18-3.63 (m, 4, H-5', O-CH₂(CH₂)₇]; 3.74 (s, 6, DMTr O-CH₃) 4.06 (m, 1, H-4'), 4 . 27 (m, 1, H-3'); 4 .42 (m, 1, H-2' ) ; 5.19 (d, 1, 3' -OH, *J*= 5 Hz), 5.94 (d, 1, H-1', *J*= 5.7 Hz), 6.83-7.38 (m, 13, DMTr aromatic), 8.14 (s, 1, 8-H), 11.65 (s, 1, C₂ amide) and 12.11 (s, 1, N₁ amide) . Anal. Calcd. for C₄₄H₅₅N₅O₈: C, 67.59; H, 7.27; N, 8.96. Found: C, 67.59; H, 7.11; N, 8.80.

### EXAMPLE 16

### N2-Isobutyryl-5'-dimethoxytrityl-2'-O-nonylguanosine 3'-β-cyanoethyl-N,N-diisopropylphosphoramidite

N2-Isobutyryl-5'-dimethoxytrityl-2'-*O*-nonylguanosine (2.1 g) was treated with bis-(N,N-diisopropylamino)-2-cyanoethyl-phosphite (1.5 g) and N,N-diisopropylammonium tetrazolide (0.2 g) as per the procedure of Example 6 to yield the product (2.0 g). ³¹P NMR (CDCl₃) δ 150.7 and 150.4 (diastereomers).

### EXAMPLE 17

### 2,6-Diamino-9-(2',3'-di-O-propyl-β-D-ribofuranosyl]purine

The procedure of Example 2 was repeated utilizing 2,6-diamino-9-(β-*D*-ribofuranosyl)purine (10 g), NaH (3 g) and 1-bromopropane (10 ml) in DMF. After evaporation of the reaction solvent, the reaction products were purified by silica gel chromatography. The slower moving component yielded 4.3 g of the 2'-*O*-propyl product as a foam. This foam was crystallized from water to yield 3.6 g of product. The faster moving component isolated as an oil formed crystals upon standing. EtOH was added to the crystals, they were filtered and wash 1 x EtOH to yield 1.1 grams of 2',3'-di-*O*-propyl product. m.p. 165-167° C. ¹H NMR (DMSO-d₆) δ 0.80 and 0.92 (t, 6, CH₃), 1.6 and 1.45 (m, 4, CH₂), 3.7-3.45 (br m, 6), 4.07 (m, 2), 4.5 (dd, 1) , 5.55 (br t, 1, 5' -OH) , 5.8 (br s, 2, 6-NH₂), 5 .85 (d, 1, H-1'), 6.84 (br s, 2, 2-NH₂) and 8.0 (s, 1, H-8).
Anal. Calcd. for C₁₆H₂₆N₆O₄: C, 52.45; H, 7.15; N, 22.94. Found: C, 52.18; H, 7.19; N, 22.75.

### EXAMPLE 18

### N2,N6-Diisobutyryl-2,6-diamiao-9-(2'-O-propyl-β-D-ribofuranosyl)purine

2,6-diamino-9-(2'-*O*-propyl-β-*D*-ribofuranosyl)purine (2.0 g) in pyridine (35 ml) was treated with trimethylsilyl chloride (3.9 ml, 5 eq) and isobutyryl chloride (3.2 ml, 5 eq) as per the procedure of Example 4 to yield a foam after silica gel chromatography. The foam was crystallized from EtOAc/Hex to yield 2.2 g of product. m.p. 140-142° C. ¹H NMR (DMSO-d₆) δ 0.77 (t, 3, CH₃), 1.07, 1.16 [d, 12, 2 x CH(CH₃)₂], 1.5 (m, 2, CH₂), 2.9, 3.03 [m, 2, 2 x CH(CH₃)₂], 3.4 (m, 1, H-5''), 3.58 (m, 3, OCH₂, H-5'), 3.95 (m, 1, H-4'), 4.3 (m, 1), 4.5 (m, 1), 5.02 (t, 1, 5'-OH), 5.2 (d, 1, 3'-OH), 6.03 (d, 1, H-1'), 8.58 (s, 1, H-8), 10.39 (br s, 1, NH), and 10.57 (br s, 1, NH).

### EXAMPLE 19

### N2,N6-Diisobutyryl-2,6-diamino-9-(5'-O-dimethoxytrityl-2'-O-propyl-β-D-ribofuranosyl)purine

N2,N6-Diisobutyryl-2,6-diamino-9-(2'-*O*-propyl-β-*D*-ribo-furanosyl)purine (1.9 g) was treated with dimethoxytrityl chloride (1.5 g, 1.1 eq), and dimethylaminopyridine (20 mg as a catalyst) in pyridine (50 ml) as per the procedure of Example 5 to yield the product as a foam (2.8 g) . ¹H NMR (DMSO-d₆) δ 0.79 (t, 3, CH₃), 1.07, 1.16 [d, 12, 2 x CH(CH₃)₂], 1.5 (m, 2, CH₂), 2.9, 3.03 [m, 2, 2 x CH(CH₃)₂], 3.58 (m, 3, OCH₂, H-5'), 4.15 (m, 1, H-4'), 4.4 (m, 1), 4.6 (m, 1), 5.15 (d, 1, 3'-OH), 6.15 (d, 1, H-1'), 6.
8-7.35 (m, 13, DMTr), 8.5 (s, 1, H-8), 10.3 (br s, 1, NH), and 10.57 (br s, 1, NH).

### EXAMPLE 20

### N2,N6-Diisobutyryl-2,6-diamino-9-(5'-O-dimethoxytrityl-2'-O-propyl-β-D-ribofuranosyl)purine 3'-β-cyanoethyl-N,N-diisopropylphoaphoramidite

N2,N6-Diisobutyryl-2,6-diamino-9-(5'-*O*-dimethoxytrityl-2'-*O*-propyl-β-*D*-ribofuranosyl)purine (2.6 g) was treated with *bis*-(N,N-diisopropylamino)-2-cyanoethylphosphite (1.7 g) and N,N-diisopropylammonium tetrazolide (300 mg) overnight at room temperature. The reaction mixture was partitioned against dil. Na₂CO₃/CHCl₂ and then Na₂CO₃/NaCl and dried over MgSO₄. The organic layer was evaporated to a foam. The foam was dissolved in CH₂Cl2 (≈8 ml) and slowly added to Hexanes (500 ml). The solid was filtered and dried to yield the product as a powder (3.1 g). ³¹P NMR (CDCl₃) δ 150.8 and 151.3.

### EXAMPLE 21

### 2,6-Diamino-9-[2'-O-[(N-phthalimido)prop-3-yl]-β-D-ribofuranosyl] purine & 2,6-Diamino-9-[3'-O-[(N-phthalimido)prop-3-yl]-β-D-ribo-furanosyl]purine

2,6-Diamino-9-(β-*D*-ribofuranosyl)purine (14.2 g) was treated with sodium hydride (3 g, 1. 5 eq) and N-(3-bromopropyl) phthalimide (5.3 ml, 1.5 eq) in DMF (20 g) at 70° C overnight. The reaction mixture was proportioned between H₂O and Hexanes (1x) and the aqueous layer then extracted 4 x CH₂Cl₂. The organic layer was dried over MgSO₄ and evaporated to a residue. The residue was purified by silica gel chromatography eluted with MeOH/CH₂Cl₂. The 2'-*O*-(N-phthalimido)propyl product eluted first followed by mixed fractions and then the 3'-*O*-(N-phthalimido) product. Evaporations of the fractions gave 3.4 g of the 2'-*O*-(N-phthalimido)propyl product, 3.0 g of mixed 2' and 3' products and 1.4 g of the 3'-*O*-(N-phthalimido)propyl product all as foams. The 3'-*O*-(N-phthalimido)propyl product was crystallized from EtOAc/MeOH to give 270 mg of solid.

### 2,6-Diamino-9-[2'-O-[(N-phthalimido)prop-3-yl]-β-D-ribofuranosyl] purine

¹H NMR (DMSO-d₆) δ 1.8 (tq, 2, -CH₂-), 3.4-3.58 (m, 6, 2x CH₂, H-5'), 3.9 (m, 1), 4.26 (m, 1), 4.37 (m, 1), 5.05 (br d, 1, 3'-OH), 5.4 (br t, 1, 5'-OH), 5.72 (br s, 2, NH₂), 5.8 (br d, 1, H-1'), 6.75 (br s, 2, NH₂), 7.8 (br s, 4, Ar) and 8.93 (s, 1, H-8).

### 2,6-Diamino-9-[3'-O-[(N-phthalimido)prop-3-yl]-β-D-ribofuranosyl] purine

m.p. 220-222° C, ¹H NMR (DMSO-d₆) δ 1.85 (tq, 2, -CH-N), 3.6-3.67 (m, 4, -O-CH₂, H-5'), 3.85 (m, 1), 3.92 (m, 1), 4.6 (m, 1), 5.33 (d, 1, 2'-OH), 5.45 (br t, 1, 5'-OH), 5.65 (d, 1, H-1'), 5.73 (br s, 2, NH₂), 6.75 (br d, 2, NH₂), 7.8-7.85 (m, 4, Ar) and 7.85 (s, 1, H-8). Anal. Calcd. for C₂₁H₂₃N₇O₆: C, 53.73; H, 4.94; N, 20.88. Found: C, 53.59; H, 4.89; N, 20.63.

### EXAMPLE 22

### 2'-O-[(N-Phthalimido)prop-3-yl] guanosine

2,6-diamino-9-[2'-*O*-[(N-phthalimido)prop-3-yl]-β-*D*-ribofuranosyl] purine (3.1 g) in 0.1 M sodium phosphate buffer (3 ml, pH 7.4), 0.05 M tris buffer (65 ml, pH 7.4) and DMSO (45 ml) was treated with adenosine deaminase (200 mg) at room temperature for 5 days as per the procedure of Example 3. The product containing fractions from the silica gel chromatography were evaporated and upon concentration formed white crystals. The crystals were filtered and washed with MeOH to yield 1.1 g of product. An analytical sample was recrystallized from MeOH. m.p. 192-194° C. ¹H NMR (DMSO-d₆) δ 1.82 (m, 2, CH₂), 3.45-3.67 (m, 6, H-5', OCH₂, NCH₂), 3.9 (m, 1), 4.3 (m, 2, H-2', H-3'), 5.1 (m, 2, 5' and 3'-OH), 5.8 (d, 1, H-1'), 6.5 (br s, 2, NH₂), 7.83 (s, 4, phthal), 7.98 (s, 1, H-8) and 10.5 (br s, 1, NH). Anal. Calcd. for C₂₁H₂₂N₆O₇·½H₂O: C, 52.61; H, 4.83; N, 17.53. Found: C, 52.52; H, 4.78; N, 17.38.

### EXAMPLE 23

### N2-Isobutyryl-2'-O-[(N-phthalimido)prop-3-yl] guanosine

2'-*O*-[(N-phthalimido)prop-3-yl] guanosine (7.2 g, crude) in pyridine (35 ml) was treated with trimethylsilyl chloride (11.6 ml, 5 eq) and isobutyryl chloride (8 ml, 5 eq) as per the procedure of Example 4 to yield the product as a crude foam (6.5 g). An analytical sample was obtained by crystallization from EtOAc. m.p. 166-168° C. ¹H NMR (DMSO-d₆) δ 1.15 [d, 6, -CH(CH₃)₂], 1.85 (m, 2, CH₂), 2.8 [m, 1, CH(CH₃)₂], 3.45-3.7 (m, 6, H-5', OCH₂, NCH₂), 3.95 (m, 1), 4.34 (m, 1), 4.4 (m, 1), 5.12 (t, 1, 5'-OH), 5.18 (d, 1, 3'-OH), 5.9 (d, 1, H-1'), 7.83 (s, 4, phthal), 8.3 (s, 1, H-8), 11.65 (br s, 1, NH) and 12.1 (br s, 1, NH). Anal. Calcd. for C₂₅H₂₈N₆O₈·½H₂O: C, 54.64; H, 5.32; N, 15.29. Found: C, 54.46; H, 5.39; N, 14.98.

### EXAMPLE 24

### N2-Isobutyryl-5'-dimethoxytrityl-2'-O-[(N-phthalimido)prop-3-yl]guanosine

N2-Isobutyryl-2'-*O*-[(N-phthalimido)prop-3-yl] guanosine (1.2 g) was treated with dimethoxytrityl chloride (820 mg, 1.1 eq), and dimethylaminopyridine (20 mg as a catalyst) in pyridine (50 ml) as per the procedure of Example 5 utilizing 1:1 Hex/EtOAc, then EtOAc then 5%MeOH/EtOAc with 1% TEA as eluent. The product containing fraction were evaporated to yield the product as a foam (1.7 g). ¹H NMR (DMSO-d₆) δ 1.1 [d, 6, -CH(CH₃)₂], 1.85 (m, 2, CH₂), 2.75 [m, 1, CH(CH₃)₂], 3.45-3.7 (m, 6, H-5' , OCH₂, NCH₂), 3.75 (s, 6, OCH₃), 4.0 (m, 1), 4.32 (m, 1), 4.4 (m, 1), 5.2 (d, 1, 3'-OH), 5.93 (d, 1, H-1'), 6.83, 7.2, 7.35 (m, 13, DMTr), 7.78 (s, 4, phthal), 8.15 (s, 1, H-8), 11.6 (br s, 1, NH) and 12.05 (br s, 1, NH). Anal. Calcd. for C₄₆H₄₆N₆O₁₀·H₂O: C, 64.18; H, 5.62; N, 9.76. Found: C, 64.42; H, 5.78; N, 9.53.

### EXAMPLE 25

### N2-Isobutyryl-5'-dimethoxytrityl-2'-O-[(N-phthalimido)prop-3-yl]guanosine 3'-β-cyanoethyl-N,N-diisopropylphosphoramidite

N2-Isobutyryl-5'-dimethoxytrityl-2'-*O*-[(N-phthalimido) prop-3-yl] guanosine (1.6 g) was treated with *bis*-(N,N-diisopropylamino)-2-cyanoethylphosphite (1.48 g) and N,N-diisopropylammonium tetrazolide (200 mg) as per the procedure of Example 6 to yield the product (2.0 g). ³¹P NMR (CDCl₃) δ 150.9.

### EXAMPLE 26

### N2-Dimethylaminomethylidene-5'-dimethoxytrityl-2'-O-[(N-phthalimido)prop-3-yl] guanosine

2'-*O*-[(N-phthalimido)prop-3-yl] guanosine (900 mg) in DMF (20 ml) was treated with N,N-dimethylformamide dimethyl acetal (2 ml). The reaction mixture was stirred for 2 hr and evaporated under high vac at 52° C. The residue was co-evaporated 1 x with pyridine and taken up in solution in pyridine. Dimethoxytrityl chloride (713 mg, 1.1 eq) and dimethylaminopyridine (20 mg as a catalyst) were added. The reaction mixture was stirred overnight, partitioned between Na₂CO₃/CH₂Cl₂, dried over MgSO₄ and purified by silica gel chromatography as per the procedure of Example 5 to yield 1.7 g of product as an off white solid. ¹H NMR (DMSO-d₆) δ 1.88 (m, 2, CH₂), 3.1 [d, 6, N=CHN(CH₃)₂], 3.3 (m, 2, H-5'), 3.67 (m, 4, OCH₂, NC₂), 3.78 (s, 6, 2x OCH₃), 4.0 (m, 1, H-4'), 4.35 (m, 2, H-2', H-3'), 5.2 (d, 1, 3'-OH), 5.95 (d, 1, H-1'), 6.85, 7.25, 7.39 (m, 13, DMTr), 7.85 (s, 4, phthal), 7.95 [s, 1, H-8), 8.5 (s, 1, N=CHN(CH₃)₂] and 11.39 (s, 1, NH₂). Anal. Calcd. for C₄₅H₄₅N₇O₉·½H₂O: C, 64.58; H. 5.54; N, 11.71. Found: C, 64.10; H, 5.65; N, 11.47.

### EXAMPLE 27

### N2-Dimethylaminomethylidene-5'-dimethoxytrityl-2'-O-[(N-phthalimido)prop-3-yl] guanosine 3'-β-cyanoethyl-N,N-diisopropylphosphoramidite

N2-dimethylaminomethylidene-5'-dimethoxytrityl-2'-*O*-[(N-phthalimido)prop-3-yl] guanosine (1.7 g), *bis*-(N,N-diisopropylamino)-2-cyanoethylphosphite (1.4 ml) and N,N-diisopropylammonium tetrazolide (170 mg) were stirred overnight at room temperature. The reaction mixture was partitioned between CH₂Cl₂ and Na₂CO₃ (2 x). The organic phase was dried over MgSO₄ and evaporated to an oil. The oil was dissolved in a minimum of CH₂Cl₂ and added dropwise to ≈ 900 ml Hexanes to precipitate the product. The solid was isolated and dried to yield 2.1 g of product. ¹P NMR (CDCl₃) δ 150.4, 150.6.

### EXAMPLE 28

### 2,6-Diamino-9-[2'-O-(N-phthalimido)pent-5-yl]-β-D-ribofuranosyl] purine

2,6-Diamino-(9-β-D-ribofuranosyl)purine (6.7 g) was treated with sodium hydride (1.3 g) and N-(5-bromopentyl) phthalimide (7.8 g, 1.1 eq) in DMF (60 ml) at room temperature for three days. The reaction mixture was proportioned between H₂O and CH₂Cl₂ and extracted 4 x CH₂Cl₂. The combined organic layers were dried over MgSO₄ and evaporated. The residue was purified by silica gel chromatography eluted with 5 → 10% MeOH/CH₂Cl₂. The 2'-*O*-(N-phthalimido)pentyl containing fractions were collected and evaporated to a yellow foam to give 2.2 g of product. An analytical sample was crystallized from EtOH. m.p. 173-175° C. ¹H NMR (DMSO-d₆) δ 1.2 (m, 2, -CH₂-), 1.47 (m, 4, 2x CH₂), 3.55, 3.65 (m, 6, O-CH₂, H-5', NCH₂), 3.95 (m, 1), 4.28 (m, 1), 4.4 (m, 1), 5.13 (d, 1, 3'-OH), 5.5 (t, 1, 5'-OH), 5.77 (br s, 2, 6-NH₂), 5.84 (br d, 1, H-1'), 6.8 (br s, 2, 2-NH₂), 7.86 (M, 4, phthal) and 7.95 (s, 1, H-8). Anal. Calcd. for C₂₃H₂₇N₇O₆: C, 55.50; H, 5.47; N, 19.71. Found: C, 55.44; H, 5.51; N, 19.30.

### EXAMPLE 29

### 2'-O-[(N-Phthalimido)pent-5-yl] guanosine

A mixture of the 2,6-diamino-9-[2'-*O*-[(N-phthalimido) pent-5-yl]-β-*D*-ribofuranosyl]purine and 2,6-diamino-9-[3'-*O*-[(N-phthalimido) pent-5-yl]-β-*D*-ribofuranosyl]purine isomers (2.2 g) in 0.1 M tris buffer (60 ml, pH 7.4), 0.1 M NaPO₄ buffer (2 ml, pH 7.4) and DMSO (40 ml) was treated with adenosine deaminase (60 mg) at room temperature for 5 days as per the procedure of Example 3. The product containing fractions from the silica gel chromatography were evaporated to give the product (1.0 g) as a crude white solid. An analytical sample was prepared by the addition of MeOH to form crystals. m.p. 178-180° C. ¹H NMR (DMSO-d₆) δ 1.24 (m, 2, CH₂), 1.5 (m, 4, 2x CH₂), 3.5-3.6 (m, 6, H-5', OCH₂, NCH₂), 3.87 (m, 1, H-4'), 4.25 (m, 2, H-2', H-3'), 5.1 (m, 2, 5' and 3'-OH), 5.78 (d, 1, H-1'), 6.5 (br s, 2, NH₂), 7.84 (M, 4, phthal), 7.98 (s, 1, H-8) and 10.67 (br s, 1, NH). Anal. Calcd. for C₂₃H₂₆N₆O₇·½H₂O: C, 54.43; H, 5.36; N, 16.56. Found: C, 54.79; H, 5.24; N, 16.61.

### EXAMPLE 30

### N2-Isobutyryl-2'-O-[(N-phthalimido)pent-5-yl] guanosine

2'-*O*-[(N-phthalimido)pent-5-yl] guanosine (1.6 g, crude) in pyridine (35 ml) was treated with trimethylsilyl chloride (2.0 ml, 5 eq) and isobutyryl chloride (1.68 ml, 5 eq) as per the procedure of Example 4 to yield the product as a foam. This foam was co-evaporated 2 x with EtOAc followed by the addition of EtOAc and heating to yield white crystals (950 mg). m.p. 202-204°C. ¹H NMR (DMSO-d₆) δ 1.1 [d, 6, -CH(CH₃)₂], 1.17 (m, 2, CH₂), 1.43 (m, 4, 2x CH₂), 2.74 [m, 1, CH(CH₃)₂], 3.45-3.55 (m, 6, H-5' , OCH₂, NCH₂), 3.9 (m, 1), 4.25 (m, 1), 4.3 (m, 1), 5.07 (t, 1, 5'-OH), 5.15 (d, 1, 3'-OH), 5.87 (d, 1, H-1'), 7.8 (s, 4, phthal), 8.27 (s, 1, H-8), 11.67 (br s, 1, NH) and 12.06 (br s, 1, NH). Anal. Calcd. for C₂₇H₃₂N₆O₈·½H₂O: C, 56.14; H, 5.76; N, 14.55. Found: C, 56.45; H, 5.74; N, 14.41.

### EXAMPLE 31

### N2-Isobutyryl-5'-dimethoxytrityl-2'-O-[(N-phthalimido)pent-5-yl] guanosine

N2-Isobutyryl-2'-*O*-[(N-phthalimido)pent-5-yl] guanosine (0.95 g) was treated with dimethoxytrityl chloride (620 mg, 1.1 eq), and dimethylaminopyridine (20 mg as a catalyst) in pyridine (50 ml) as per the procedure of Example 5 utilizing EtOAc 1% TEA and then 5% MeOH EtOAc/CH₂Cl₂ with 1% TEA as eluent. The product containing fractions were evaporated to yield the product as a foam (1.4 g). ¹H NMR (DMSO-d₆) δ 1.14 [d, 6, -CH(CH₃)₂], 1.25 (m, 2, CH₂), 1.53 (m, 4, 2x CH₂), 2.77 [m, 1, CH(CH₃)₂], 3.3-3.6 (m, 6, H-5' , OCH₂, NCH₂), 3.75 (s, 6, OCH₃), 4.07 (m, 1), 4.33 (m, 1), 4.4 (m, 1), 5.18 (d, 1, 3'-OH), 5.94 (d, 1, H-1'), 6.83, 7.2, 7.53 (m, 13, DMTr), 7.8 (s, 4, phthal), 8.15 (s, 1, H-8), 11.6 (br s, 1, NH) and 12.1 (br s, 1, NH). Anal. Calcd. for C₄₈H₅₀N₆O₁₀·½H₂O: C, 65.52; H, 5.84; N, 9.55. Found: C, 65.55; H, 5.94; N, 9.20.

### EXAMPLE 32

### 2,6-Diamino-9-[3',5'-O-(tetraisopropyldisiloxane-1,3-diyl)-β-D-ribofuranosyl]purine

To a suspension of 2,6-diamino-9-(β-*D*-ribofuranosyl)purine (10.5 g) in pyridine (100 ml) was added 1,3-dichlorotetraisopropyldisiloxane (TIPDS, 12.6 g). The reaction was stirred at room temperature for 4 hours and an additional 1.3 g of 1,3-dichlorotetraisopropyldisiloxane was added followed by stirring overnight. The reaction mixture was poured into ice water and the insoluble product (11.6 g) collected by filtration. An analytical sample was recrystallized from EtOAc/Hexanes. m.p. 170-172° C. Anal. Calcd. for C₂₂H₄₀N₆O₅Si₂·½H₂O: C, 49.5; H, 7.74; N, 15.7. Found: 49.57; H, 7.82; N, 15.59.

### EXAMPLE 33

### 2,6-Diamino-9-[3',5'-O-(tetraisopropyldisiloxane-1,3-diyl)-2-O-methyl-β-D-ribofuranosyl] purine

A mixture of 2,6-Diamino-9-[3,5-*O*-(tetraisopropyldisiloxane-1,3-diyl)-β-*D*-ribofuranosyl]purine (8.8 g) in DMF (120 ml) and methyl iodide (3 ml, 3 eq) was cooled in an ice bath and NaH (60% in oil, 1.0 g, 1.5 eq) added. After 20 min the reaction was quenched with MeOH and partitioned between sat. NH₄Cl and CH₂Cl₂. The organic phase was washed with 1 x NH₄Cl, dried over MgSO₄ and evaporated. The residue was crystallized from hot EtOH/H₂O to yield the product (8.5 g) as crystals. m.p. 87-89°C. ¹H NMR (DMSO-d₆) δ 1.05 (m, 28, TIPDS), 3.57 (s, 3, OCH₃), 3.98 (m, 1, H-4'), 3.92 and 4.07 (ABX, 2, H-5'), 4.13 (d, 1), 4.6 (dd, 1, H-3'), 5.76 (br s, 2, NH₂), 5.8 (s, 1, H-1'), 6.77 (br s, 2, NH₂) AND 7.77 (s, 1 H-8).

### EXAMPLE 34

### 2,6-Diamino-9-(2'-O-methyl-β-D-ribofuranosyl)purine

To a solution of 2,6-Diamino-9-[3',5'-*O*-(tetraisopropyldisiloxane-1,3-diyl)-2'-*O*-methyl-β-*D*-ribofuranosyl]purine (8.5 g) in THF (50 ml) was added 1M tetrabutylammonium fluoride in THF (Aldrich, 20 ml). The reaction mixture was stirred for 2 hrs and filtered. The filter cake was washed with 2 x EtOAc and air dried to give 4.0 g of crude product. An analytical sample was crystallized from hot MeOH. m.p. 133-135° C. ¹H NMR (DMSO-d₆) δ 3.3 (s, 3, OCH₃), 3.58 (m, 2, H-5'), 3.98 (m, 1, H-4'), 4.28 (m, 2, H-2', H-3'), 5.23 (br s, 1, 3'-OH), 5.48 (br t, 1, 5'-OH), 5.77 (br s, 2, NH₂), 5.82 (d, 1, H-1'), 6.83 (br s, 2, NH₂) and 7.95 (s, 1, H-8). Anal. Calcd. for C₁₁H₁₆N₆O₄·½H₂O: C, 43.28; H, 5.61; N, 27.52. Found: C, 43.51; H, 5.62; N, 27.26.

### EXAMPLE 35

### 2'-O-Methylguanosine

2,6-Diamino-9-(2'-*O*-methyl-β-*D*-ribofuranosyl)purine (9.5 g) in 0.1M sodium phosphate buffer (200 ml, pH 7.4) and DMSO (25 ml) was treated with adenosine deaminase (Type II Sigma) at RT for 4 days. The resulting suspension was cooled and filtered and the resulting filter cake washed with H₂O and dried to a white solid (4.0 g). The solid was recrystallized from hot H₂O to yield 2.9 g of product. m.p. 236-238° C. ¹H NMR (DMSO-d₆) δ 3.3 (s, 3, OCH₃), 3.53 and 3.6 (ABX, 2, H-5'), 3.87 (m, 1, H-4'), 4.15 (m, 1, H-2'), 4.25 (m, 1, H-3'), 5.13 (t, 1, 5'-OH), 5.23 (d, 1, 3'-OH), 5.8 (d, 1, H-1'), 6.48 (br s, 2, NH₂), 7.96 (s, 1, H-8) and 10.68 (br s, 1, NH). Anal. Calcd. for C₁₁H₁₅N₅O₅·½H₂O: C, 43.14; H, 5.26; N, 22.86. Found: C, 43.59; H, 5.34; N, 23.04.

### EXAMPLE 36

### N2-Isobutyryl-2'-O-methylguanosine

2'-*O*-methylguanosine (3.5 g) in pyridine (100 ml) was treated with trimethylsilyl chloride (9 ml, 6 eq) and isobutyryl chloride (6.2 ml) at RT for 4 hr. The reaction mixture was cooled in an ice bath, H₂O (20 ml) was added and stirring continued for an additional 20 min. NH₄OH (20 ml) was added and after stirring for 30 min the reaction mixture was evaporated. The residue was triturated with H₂O, filtered and the filtrate evaporated and purified by silica gel chromatography as per the procedure of Example 4 to yield the product as an off white solid (1.5 g). ¹H NMR (DMSO-d₆) δ 1.1 [d, 6, CH(CH₃)₂], 2.77 [m, 1, CH(CH₃)₂], 3.33-3.6 (m, 5, OCH₃, H-5'), 3.93 (m, 1, H-4'), 4.22 (m, 1), 4.3 (m, 1), 5.1 (t, 1, 5'-OH), 5.28 (d, 1, 3'-OH), 5.9 (d, 1, H-1'), 8.28 (s, 1, H-8) and 11.9 (br s, 1, NH).

### EXAMPLE 37

### N2-Isobutyryl-5'-dimethoxytrityl-2'-O-methylguanosine

N2-isobutyryl-2'-*O*-methylguanosine (1.5 g) was treated with dimethoxytrityl chloride (1.5 g, 1.1 eq), and dimethylaminopyridine (100 mg as a catalyst) in pyridine (50 ml) as per the procedure of Example 5 to yield the product as a foam (2.6 g). ¹H NMR (DMSO-d₆) δ 1.14 (d, 6, CH(CH₃)₂], 2.75 [m, 1, CH(CH₃)₂], 3.5 (m, 2, H-5'), 3.74 (s, 6, OCH₃), 4.05 (m, 1), 4.33 (m, 1), 5.26 (d, 1, 3'-OH), 5.95 (d, 1, H-1'), 6.83, 7.2, 7.35 (m, 13, DMTr), 8.15 (s, 1, H-8), 11.6 (br s, 1, NH) and 12.1 (br s, 1, NH).

### EXAMPLE 38

### N2-Isobutyryl-5'-dimethoxytrityl-2'-O-methylguanosine 3'-β-cyanoethyl-N,N-diisopropylphosphoramidite

N2-Isobutyryl-5'-dimethoxytrityl-2'-*O*-methylguanosine (20 g) was treated with *bis*-(N,N-diisopropylamino)-2-cyanoethylphosphite (10.8 g) and N,N-diisopropylammonium tetrazolide (1.6 g) as per the procedure of Example 6 to yield the product (15.7 g). ³¹P NMR (CDCl₃) δ 148.97 and 147.96.

### EXAMPLE 39

### N2,N6-Diisobutyryl-2,6-diamino-9-(2'-O-methyl-β-D-ribofuranosyl) purine

2,6-diamino-9-(2'-*O*-methyl-β-*D*-ribofuranosyl)purine (700 mg) in pyridine (20 ml) was treated with trimethylsilyl chloride (2.1 ml, 7 eq) and isobutyryl chloride (1.25 ml, 5 eq) as per the procedure of Example 4 to yield the product as a foam (900 mg) after silica gel chromatography.

### EXAMPLE 40

### N2,N6-Diisobutyryl-2,6-diamino-9-(5'-O-dimethoxytrityl-2'-O-methyl-β-D-ribofuranosyl)purine

N2,N6-Diisobutyryl-2,6-diamino-9-(2'-*O*-methyl-β-*D*-ribofuranosyl)purine (900 mg) was treated with dimethoxytrityl chloride (1.0 g) and dimethylaminopyridine (20 mg as a catalyst) in pyridine (30 m) as per the procedure of Example 5 to yield the product as a foam (700 mg). ¹H NMR (DMSO-d₆) δ 0.96-1.16 [m, 12, 2x CH(CH₃)₂], 2.9 and 3.05 [M, 2, 2x CH(CH₃)₂], 3.18 and 3.37 (ABX, 2, H-5'), 3.38 (s, 3, OCH₃), 3.7 (s, 6, OCH₃), 4.05 (m, 1, H-4'), 4.44 (m, 2, H-2',H-3'), 5.24 (d, 1, 3'-OH), 6.06 (d, 1, H-1'), 6.78, 7.2, 7.33 (m, 13, Ar), 8.22 (s, 1, H-8), 10.3 (br s, 1, NH) and 10.57 (br s, 1, NH).

### EXAMPLE 41

### N2,N6-Diisobutyryl-2,6-diamino-9-(5'-O-dimethoxytrityl-2'-O-methyl-β-D-ribofuranosyl)purine 3'-β-cyanoethyl-N,N-diisopropylphosphoramidite

N2,N6-Diisobutyryl-2,6-diamino-9-(5'-*O*-dimethoxytrityl-2'-*O*-methyl-β-*D*-ribofuranosyl)purine (600 mg) was treated with *bis*-(N,N-diisopropylamino)-2-cyanoethylphosphite (500 µl) and N,N-diisopropylammonium tetrazolide (80 mg) overnight at RT. The reaction mixture was partitioned against dil. Na₂CO₃/CHCl₂ and then Na₂CO₃/NaCl and dried over MgSO₄. The organic layer was evaporated to a foam (500 mg). ³¹P NMR (CDCl₃) δ 151.1 (doublet).

### EXAMPLE 42

### 2,6-Diamino-9-(2'-O-octadecyl-β-D-ribofuranosyl)purine

2,6-Diamino-9-(β-*D*-ribofuranosyl)purine (50 g, 180 mmol) and sodium hydride (7 g) in DMF (1 l) were heated to boiling for 2 hr. Iodooctadecane (100 g) was added at 150° C and the reaction mixture allowed to cool to RT. The reaction mixture was stirred for 11 days at RT. The solvent was evaporated and the residue purified by silica gel chromatography. The product was eluted with 5% MeOH/CH₂Cl₂. The product containing fraction were evaporated to yield the product (11 g). ¹H NMR (DMSO-d₆) δ 0.84 (t, 3, CH₂); 1.22 [m, 32, O-CH₂-CH₂-(CH₂)₁₆-]; 1.86 (m, 2, O-CH₂CH₂-); 3.25 (m, 2, O-CH₂-); 3.93 (d, 1, 4'H), 4.25 (m, 1, 3'H); 4.38 (t, 1, 2'H); 5.08 (d, 1, 3'-OH); 5.48 (t, 1, 5'-OH); 5.75 (s, 2, 6-NH₂); 5.84 (d, 1, 1'-H); 6.8 (s, 2, 2-NH₂); and 7.95 (s, 1, 8-H).

### EXAMPLE 43

### 2'-O-Octadecylguanosine

2,6-Diamino-9-(2'-*O*-Octadecyl-β-*D*-ribofuranosyl) purine (10 g) in 0.1 M sodium phosphate buffer (50 ml, pH 7.4), 0.1 M tris buffer (1000 ml, pH 7.4) and DMSO (1000 ml) was treated with adenosine deaminase (1.5 g) as per the procedure of Example 3. At day 3, day 5 and day 7 an additional aliquot (500 mg, 880 mg and 200 mg, respectively) of adenosine deaminase was added. The reaction was stirred for a total of 9 day and after purification by silica gel chromatography yielded the product (2 g). An analytical sample was recrystallized from MeOH ¹H NMR (DMSO-d₆) δ 0.84 (t, 3, CH₃), 1.22 [s, 32, O-CH₂-CH₂-(CH₂)₁₆], 5. 07 (m, 2, 3' -OH 5' -OH); 5.78 (d, 1, 1'-H); 6.43 (s, 2, NH₂), 7.97 (s, 1, 8-H) and 10.64 (s, 1, NH₂). Anal. Calcd. for C₂₈H₄₉N₅O₅: C, 62.80; H, 9.16; N, 12.95. Found: C, 62.54; H, 9.18; N, 12.95.

### EXAMPLE 44

### N2-Isobutyryl-2'-O-octadecylguanosine

2'-*O*-Octadecylguanosine (1.9 g) in pyridine (150 ml) was treated with trimethylsilyl chloride (2 g, 5 eq) and isobutyryl chloride (2 g, 5 eq) as per the procedure of Example 4. The product was purified by silica gel chromatography (eluted with 3% MeOH/EtOAc) to yield 1. 2 g of product. ¹H NMR (DMSO-d₆) δ 0. 85 [t, 3, CH₃], 1.15 [m, 38, O-CH₂CH₂(CH₂)₁₆, CH(CH₃)₂], 2.77 [m, 1, CH(CH₃)₂], 4.25 (m, 2, 2'H, 3'H); 5.08 (t, 1, 5'-OH), 5.12 (d, 1, 3'-OH), 5.87 (d, 1, 1'-H), 8.27 (s, 1, 8-H), 11.68 (s, 1, NH₂) and 12.08 (s, 1, NH₂). Anal. Calcd. for C₃₂H₅₅N₅O₆: C, 63.47; H, 9.09; N, 11.57. Found: C, 63.53; H, 9.20; N, 11.52.

### EXAMPLE 45

### 2,6-Diamino-9-[2'-O-(imidazol-1-yl)butyl-β-D-ribofuranosyl] purine

2,6-Diamino-(9-β-*D*-ribofuranosyl)purine (5.0 g) in DMF (400 ml) was treated with sodium hydride (0.78 g). After stirring an additional 30 min a further portion of sodium hydride (2.6 g) was added immediately followed by bromobutylimidazole (9.9 g) in DMF (25 ml). The reaction mixture was stirred overnight and quencned with H₂O. The reaction mixture was filtered through celite and evaporated to yield an oily product. TLC showed a mixture of isomers.

### EXAMPLE 46

### 2'-O-(Imidazol-1-yl)butylguanosine

A mixture of the 2,6-diamino-9-[2'-*O*-(imidazol-1-yl)butyl-β-*D*-ribofuranosyl]purine and 2,6-diamino-9-[3'-*O*-(imidazol-1-yl)butyl-β-*D*-ribofuranosyl]purine isomers in 0.1 M tris buffer (pH 7.4), 0.1 M NaSO₄ buffer (pH 7.4) and DMSO is treated with adenosine deaminase at RT for 5 days as per the procedure of Example 3. The product containing fractions are purified by silica gel chromatography and the product containing fraction evaporated to give the product.

### EXAMPLE 47

### N2-Isobutyryl-2'-O-(imidazol-1-yl)butylguanosine

2'-*O*-(imidazol-1-yl)butylguanosine in pyridine will be treated with trimethylsilyl chloride (5 eq) and isobutyryl chloride (5 eq) as per the procedure of Example 4 to yield the product.

### EXAMPLE 48

### N2-Isobutyryl-5'-dimethoxytrityl-2'-O-(imidazol-1-yl)butylguanosine

N2-Isobutyryl-2'-*O*-(imidazol-1-yl)butylguanosinewill be treated with dimethoxytrityl chloride (1.1 eq), and dimethylaminopyridine (as a catalyst) in pyridine as per the procedure of Example 5. After chromatography purification, the product containing fractions will be evaporated to yield the product).

### EXAMPLE 49 (Comparative example)

### 2',3'-O-Dibutylstannylene uridine

Utilizing the protocol of Wagner, et al., *J. Org. Chem.* **1974,** *39*, 24, uridine (45 g, 0.184 mol) was refluxed with di-n-butyltinoxide (45 g, 0.181 mol) in 1.4 l of anhydrous methanol for 4 hrs. The solvent was filtered and the resultant 2',3'-*O*-dibutylstannylene-uridine was dried under vacuum at 100°C for 4 hrs to yield 81 g (93%).

### EXAMPLE 50 (Comparative example)

### 2'-O-[Pentyl-ω-(N-phthalimido)]uridine

2',3'-*O*-Dibutyl stannylene-uridine was dried over P₂O₅ under vacuum for 12 hrs. To a solution of this compound (20 g, 42.1 mmols) in 500 ml of anhydrous DMF were added 25 g (84.2 mmols) of N(5-bromopentyl)phthalimide (Trans World Chemicals, Rockville, Maryland) and 12.75 g (85 mmols) of cesium fluoride (CsF). The mixture was stirred at room temperature for 72 hrs. The reaction mixture was evaporated then co-evaporated once with toluene and the residue was partitioned between EtOAc and water (400 ml each). The EtOAc layer was concentrated and applied to a silica column (700 g). Elution with CH₂Cl₂-CH₃OH (20:1, v/v) gave fractions containing a mixture of the 2'- and 3'- isomers of *O*-pentyl-ω-N-phthalimido uridine, in 50% yield.

### EXAMPLE 51 (Comparative example)

### 5'-O-Dimethoxytrityl-2'-O-[pentyl-ω-(N-phthalimido)]uridine

The isomeric mixture of 2'-*O*-[pentyl-ω-(N-phthalimido)]uridine was allowed to react with DMT chloride in dry pyridine at room temperature for 6 hrs. CH₃OH was used to quench excess DMT-Cl and the residue was partitioned between CH₂Cl₂ containing 0.5% Et₃N and water. The organic layer was dried (MgSO₄) and the residue was applied to a silica column. The column was eluted with CH₂Cl₂:CH₃OH (20:1, v/v) to separate the 2' and 3' isomers of the product.

### EXAMPLE 52 (Comparative example)

### 5'-O-Dimethoxytrityl-2'-O-[pentyl-ω-(N-phthalimido)]uridine-3'-O-(β-cyanoethyl N,N-diisopropylphosphoramidite)

5'-*O*-Dimethoxytrityl-2'-*O*-[pentyl-ω-(N-phthalimido)]uridine was placed in a dry round bottom flask containing a teflon stir-bar. The flask was purged with argon. Anhydrous methylene chloride was added to the flask in an amount sufficient to dissolve the nucleoside. Previously vacuum dried N,N-diisopropylaminohydrotetrazolide (600 mg, 0.014 mol) was added under argon. Bis-N,N-diisopropylaminocyanoethylphosphite was added via syringe. The reaction was stirred under argon at 25°C for 16 h. Upon completion of the reaction, the reaction was transferred to a separatory funnel. The reaction flask was rinsed with methylene chloride (2x50 mL). The combined organic layer was washed 2 x with sat'd aq. sodium bicarbonate. The organic layer was dried over magnesium sulfate, evaporated and taken up in toluene containing 1% triethylamine. The resulting phosphoramidite was purified by silica gel flash chromatography and eluted with 3:1 → 1:1 Hexanes/ethyl acetate containing 1% triethylamine. Selected fractions were combined, concentrated under reduced pressure and dried to yield the product as a white foam. ³¹P-NMR (CDCl₃, H₃PO₄ std.) showed the correct diastereomers

### EXAMPLE 53 (Comparative example)

### 2'-O-Pentyluridine

Utilizing the procedures of Examples 50 and 51, 2',3'-*O*-dibutylstannylene uridine (19.1 g) was treated with bromopentane (7 ml, 1.3 eq.) and sodium iodide (4.5 g) in DMF (90 ml). Purification on a silica gel column utilizing MeOH/CH₂Cl₂ 5% → 10% yielded the a mixture of 2' and 3' isomers of the product as a dark oil (9.8 g).

### EXAMPLE 54 (Comparative example)

### 5'-O-Dimethoxytrityl-2'-O-pentyluridine

The mixture of 2'-*O*-pentyluridine and 3'-*O*-pentyluridine (9.8 g) was reacted with dimethoxytrityl chloride (10.5 g) as per the procedure of Example 51. The crude product was purified on a silica gel column (1000 g). Elution with Hex.-EtOAc (3:1 → 1:1) gave 5.5 g of the 2'-*O*-pentyl isomer and 3 g of the 3'-*O*-pentyl isomer. Anal. Calcd. for C₃₅H₃₇N₂O₈·½H₂O: C, 67.51; H, 6.55; N, 4.5. Found: C, 67.48; H, 6.55; N, 4.5.

### EXAMPLE 55 (Comparative example)

### 5'-O-Dimethoxytrityl-2'-O-pentyluridine-3'-O-(β-cyanoethyl N,N-diisopropylphosphoramidite)

The protected 5'-*O*-dimethoxytrityl-2'-*O*-pentyluridine (4.6 g. 0.007 mol) was placed in a dry round bottom flask containing a teflon stir-bar. The flask was purged with argon. Anhydrous methylene chloride was added to the flask in an amount sufficient to dissolve the nucleoside. Previously vacuum dried N,N-diisopropylaminohydrotetrazolide (600 mg, 0.014 mol) was added under argon. Bis-N,N-diisopropylaminocyanoethylphosphite (4.5 g, 4.7 ml, 2 eq.) was added with stirring via syringe. The reaction was stirred under argon at 25°C for 16 h. After verifying the completion of the reaction by TLC, the reaction was transferred to a separatory funnel and the reaction flask was rinsed with methylene chloride (2x50 mL). The combined organic layer was washed 2 x with sat'd aq. sodium bicarbonate. The organic layer was dried over magnesium sulfate, evaporated and taken up in toluene containing 1% triethylamine. The resulting phosphoramidite was purified by silica gel flash chromatography (300 g) and eluted with Hexanes/ethyl acetate (3:1 → 1:1 containing 1% triethylamine). Selected fractions were combined, concentrated under reduced pressure and dried to yield 2.67 g of product as a white foam. ³¹P-NMR (CDCl₃, H₃PO₄ std.) showed the correct diastereomers

### EXAMPLE 56 (Comparative example)

### 2'-O-Methyluridine

As per the procedure of Example 49, uridine (8.5 g) was treated with dibutyl tin oxide (8.2 g, 1 eq). The resulting 2',3'-*O*-dibutylstannylene uridine was treated with iodomethane (16 ml) at 42°C as per Example 50 to give a mixture of the 2' and 3' alkylated products (3.5 g) as a foam.

### EXAMPLE 57 (Comparative example)

### 5'-O-(4,4'-Dimethoxytriphenylmethyl)-2'-O-methyluridine

2'-*O*-Methyluridine (8.0 g, 0.031 mol) was evaporated under reduced pressure with pyridine (100 mL) to an oil. To the residue was added 4,4'-dimethoxytriphenylmethyl chloride (DMT-Cl, 11.5 g, 0.34 mol) and pyridine (100 mL). The mixture was stirred at 25°C for 1.5 h and then quenched by the addition of methanol (10 mL) for 30 min. The mixture was concentrated under reduced pressure and the residue was chromatographed on silica gel (250 g), Elution with hexanes-ethyl acetate-triethylamine (80:20:1) and then ethyl acetate-triethylamine (99:1). The appropriate fractions were combined, evaporated under reduced pressure and dried at 25°C/0.2 mmHg for 1 h to provide 17.4 g (100 %) of tan foam; TLC purity 98% (Rf 0.23, hexanes-ethyl acetate 4:1); PMR (DMSO) d 11.4 (H-N³), 7.78 (H-6), 7.6-6.8 (Bz), 5.8 (H-1'), 5.3 (H-5'), 5.25 (HO-3'), 3.7 (CH₃O-Bz), 3.4, (CH₃O-2').

### EXAMPLE 58 (Comparative example)

### 5'-O-(4,4'-dimethoxytriphenylmethyl)-2'-O-methyluridine-3'-O-(β-cyanoethyl N,N-diisopropylphosphoramidite)

The product was prepared as per Example 54 from the intermediate5'-*O*-(4,4'-dimethoxytriphenylmethyl)-2'-*O*-methyluridine. Ethyl acetate-hexanes-triethylamine (59:40:1) was used as the chromatography eluent to give the product as a solid foam in 60% yield. TLC homogenous diastereomers, Rf 0.58; 0.44 [ethyl acetate-hexanes-triethylamine 59:40:1)]. ³¹P-NMR (CDCl₃, H₃PO₄ std.) d 148.11; 148.61 (diastereomers).

### EXAMPLE 59 (Comparative example)

### 2'-O-Propyluridine

As per the procedure of Example 49, uridine (10 g) was treated with dibutyl tin oxide (10.2 g, 1 eq). The resulting 2',3'-*O*-dibutylstannylene uridine was treated with iodopropane (8 ml, 2 eq.) at 110°C as per Example 50 to give a mixture of the 2' and 3' isomers (5.5 g) as a foam.

### EXAMPLE 60 (Comparative example)

### 5'-O-(4,4'-Dimethoxytriphenylmethyl)-2'-O-propyluridine

The mixture of 2'-*O*-propyluridine and 3'-*O*-propyluridine (3.6 g) was reacted with dimethoxytrityl chloride (4.2 g, 1.0 eq.) as per Example 51. The residue was chromatographed on silica gel eluted with Hex/EtOAc (1:1 with 1% triethylamine). The appropriate fractions were combined, evaporated under reduced pressure and dried to provide 4.2 g of a white foam. Anal. Calcd. for C₃₃H₃₆N₂O₈·½H₂O: C, 67.33; H, 6.16; N, 4.76. Found: C, 67.15; H, 6.24; N, 4.44.

### EXAMPLE 61 (Comparative example)

### 5'-O-(4,4'-dimethoxytriphenylmethyl)-2'-O-propyluridine-3'-O-(β-cyanoethyl N,N-diisopropylphosphoramidite)

The product was prepared as per Example 54 from the intermediate 5'-*O*-(4,4'-dimethoxytrityl)-2'-*O*-progyluridine (470 mg) to yield the product as a foam (477 mg).

### EXAMPLE 62 (Comparative example)

### 2'-O-Nonyluridine

As per the procedure of Example 49, uridine (22.5 g) was treated with dibutyl tin oxide (22.5 g, 1 eq). The resulting 2',3'-*O*-dibutylstannyleneuridine was treated with iodononane (11 ml, 1.3 eq.) at 130-140°C as per Example 50 to give the 2' and 3' isomers (11.2 g) as an oil.

### EXAMPLE 63 (Comparative example)

### 5'-O-(4,4'-Dimethoxytriphenylmethyl)-2'-O-nonyluridine

The mixture of 2'-*O*-nonyluridine and 3'-*O*-nonyluridine (11.2 g) was reacted with dimethoxytrityl chloride (10.5 g) as per Example 51. The residue was chromatographed on silica gel eluted with Hex/EtOAc (3:1 → 1:1 with 1% triethylamine). The appropriate fractions were combined, evaporated under reduced pressure and dried to provide 5.2 g of a foam. An analytical sample was rechromatographed using toluene/EtOAc (3:1 with 1% triethylamine) Anal. Calcd. for C₃₉H₄₈N₂O₈: C, 69.62; H, 7.19; N, 4.16. Found: C, 69.66; H, 7.18; N, 4.06.

### EXAMPLE 64 (Comparative example)

### 5'-O-(4,4'-dimethoxytriphenylmethyl)-2'-O-nonyluridine-3'-O-(β-cyanoethyl N,N-diisopropylphosphoramidite)

The product was prepared as per Example 54 from the intermediate 5'-*O*-(4,4'-dimethoxytrityl)-2'-*O*-nonyluridine (3.1 g) to yield the product as a foam (2.49 g).

### EXAMPLE 65 (Comparative example)

### 2'-O-Hexenyluridine

As per the procedure of Example 49, uridine (10.5 g) was treated with dibutyl tin oxide (10.5 g, 1 eq). The resulting 2',3'-*O*-dibutylstannyleneuridine was treated with 6-bromohexene (3.5 ml, 1.2 eq.) and sodium iodide (3.3 g, 1.0 eq.) at 115°C as per Example 50 to give the 2' and 3' isomers (3.3 g) as a foam.

### EXAMPLE 66 (Comparative example)

### 5'-O-(4,4'-Dimethoxytriphenylmethyl)-2'-O-hexenyluridine

The mixture of 2'-*O*-hexenyluridine and 3'-*O*-hexenyluridine (3.1 g) was reacted with dimethoxytrityl chloride (3.5 g, 1.1 eq.) as per Example 51. The residue was chromatographed on silica gel eluted with Hex/EtOAc (3:1 → 1:1 with 1% triethylamine). The appropriate fractions were combined, evaporated under reduced pressure and dried to provide 2.3 g of a white foam. Anal. Calcd. for C₃₆H₄₀N₂O₈·½H₂O: C, 67.80; H, 6.48; N, 4.39. Found: C, 68.77; H, 6.41; N, 4.45.

### EXAMPLE 67 (Comparative example)

### 5'-O-(4,4'-Dimethoxytriphenylmethyl)-2'-O-hexenyluridine-3'-O-(β-cyanoethyl N,N-diisopropylphosphoramidite)

The product is prepared as per Example 54 from the intermediate 5'-*O*-(4,4'-dimethoxytrityl)-2'-*O*-hexenyluridine.

### EXAMPLE 68 (Comparative example)

### 5'-O-Dimethoxytrityl-2'-O-[hexyl-ω-(N-phthalimido)]uridine-3'-O-(β-cyanoethyl N,N-diisopropylphosphoramidite)

In a like manner as per Examples 50 through 52, using N-(6-bromohexyl)phthalimide, an N-phthalimide substituted hexyl group was introduced at the 2'-position of uridine followed by dimethoxytritylation and phosphitylation to give the title nucleotide.

### EXAMPLE 69 (Comparative example)

### 5'-O-Dimethoxytrityl-2-O-[decyl-ω-(N-phthalimido)]uridine-3-O-(β-cyanoethyl N,N-diisopropylphosphoramidite)

In a like manner as per Examples 50 through 52, using N-(10-bromodecyl)phthalimide, an N-phthalimide substituted decyl group was introduced at the 2'-position of uridine followed by dimethoxytritylation and phosphitylation to give the title nucleotide.

### EXAMPLE 70 (Comparative example)

### N4-Benzoyl-2'-O-methylcytidine, Method A

### Step 1. 3',5'-O-[(1,1,3,3-Tetraisopropyl)-1,3-disiloxanediyl]cytidine

With stirring, cytidine (40 g, 0.165 mol) and 1,3-dichloro-1,1,3,3-tetraisopropyldisiloxane (TIPS-Cl, 50 g, 0.159 mol) were added to dry pyridine (250 mL). After stirring for 16 h at 25°C, the reaction was concentrated under reduced pressure to an oil. The oil was dissolved in methylene chloride (800 mL) and washed with sat'd sodium bicarbonate (2x300 mL). The organic layer was passed through a silica gel (200 g) scrub column. The product was recovered by elution with methylene chloride-methanol (97:3). The appropriate fractions were combined, evaporated under reduced pressure and dried at 25°C/0.2 mmHg for 1 h to give 59.3 g (77%) of oil. TLC purity 95% (Rf 0.59, ethyl acetate-methanol 9:1). The product may be crystallized from ethyl acetate as white crystals, mp 242-244°C. 41 PMR (DMSO) d 7.7 (H-6), 5.68 ( H-5), 5.61 (HO-2'), 5.55 (H-1').

### Step 2. N4-Benzoyl-3'-5'-O-[(1,1,3,3)tetraisopropyl-1,3-disiloxanediyl]cytidine

Benzoyl chloride (18.5 g, 0.13 mol) was added over 30 min to a stirred solution of 3',5'-*O*-[(1,1,3,3-tetraisopropyl)-1,3-disiloxanediyl]cytidine (58 g, 0.12 mol) and triethylamine (15.6 g, 0.16 mol) in dimethylacetamide (400 mL) at 5°C. The mixture was allowed to warm to 25°C for 16 h and then poured onto ice water (3.5 L) with stirring. The resulting solid was collected, washed with ice water (3x500 mL) and dried at 45°C/0.2 mmHg for 5 h to provide 77 g (100%) of solid. TLC purity ca. 90% (Rf 0.63, chloroform-methanol 9:1); PMR (CDCL₃) d 8.32 (H-6); mp 100-101°C.

### Step 3. N4-Benzoyl-2'-O-methyl-3',5'-O-[(1,1,3,3)tetraisopropyl-1,3-disiloxanediyl] cytidine

A mixture of N4-benzoyl-3'-5'-*O*-[(1,1,3,3)tetraisopropyl-1,3-disiloxanediyl]cytidine (166 g, 0.25 mol, 90% purity), silver oxide (150 g, 0.65 mol) and toluene (300 mL) was evaporated under reduced pressure. More toluene (500 mL) was added and an additional amount (100 mL) was evaporated. Under a nitrogen atmosphere, methyl iodide was added in one portion and the reaction was stirred at 40°C for 16 h. The silver salts were collected and washed with ethyl acetate (3x150 mL). The combined filtrate was concentrated under reduced pressure. The residue was dissolved in a minimum of methylene chloride, applied to a silica gel column (1 kg) and eluted with hexanes-ethyl acetate (3:2 → 1:1). The appropriate fractions were combined, concentrated under reduced pressure and dried at 45°C/0.2 mmHg for 1 h to yield 111 g (66%) of oil; TLC purity ca. 90% (Rf 0.59, hexanes-ethyl acetate 3:2). PMR (CDCl₃) d 8.8 (br s, 1 , H-N⁴), 8.40 (d, 1, H-6) , 8.0-7.4 (m, 6, H-5 and Bz), 5.86 (s, 1, H-1'), 3.74 (s, 3, CH₃O-2').

### Step 4. N4-Benzoyl-2'-O-methylcytidine

A solution of N4-benzoyl-2'-*O*-methyl-3',5'-*O*-[(1,1,3,3)tetraisopropyl-1,3-disiloxanediyl]cytidine (111 g, 0.18 mol) in methanol (160 mL) and tetrahydrofuran (640 mL) was treated with tetrabutylammonium fluoride solution (368 mL, 1 M in tetrahydrofuran). The reaction was stirred at 25°C for 16 h. The pH was adjusted to 7 with Amberlite IRC-50 resin. The mixture was filtered and the resin was washed with hot methanol (2x200 mL). The combined filtrate was concentrated under reduced pressure, absorbed on silica gel (175 g) and chromatographed on silica gel (500 g, ethyl acetate-methanol 19:1 → 4:1). Selected fractions were combined, concentrated under reduced pressure and dried at 40°C/0.2 mmHg for 2 h to yield 28 g (42.4%, 21.5% from cytidine) of solid; TLC homogenous (Rf 0.37, ethyl acetate). mp 178-180°C (recryst. from ethanol); PMR (CDCl₃) d 11.22 (br s, 1, H-N⁴), 8.55 (d, 1, H-6), 8.1-7.2 (m, 6, H-5 and Bz), 5.89 (d, 1, H-1'), 5.2 (m, 2, HO-3',5'), 3.48 (s, 3, CH₃O-2').

### EXAMPLE 71 (Comparative example)

### N4-Benzoyl-2'-O-methylcytidine, Method B

### Step 1. 2'-O-methylcytidine

Cytidine (100 g, 0.41 mol) was dissolved in warm dimethylformamide (65°C, 1125 mL). The solution was cooled with stirring to 0°C. A slow, steady stream of nitrogen gas was delivered throughout the reaction. Sodium hydride (60% in oil, washed thrice with hexanes, 18 g, 0.45 mol) was added and the mixture was stirred at 0°C for 45 min. A solution of methyl iodide (92.25 g, 40.5 mL, 0.65 mol) in dimethylformamide (400 mL) was added in portions over 4 h at 0°C. The mixture was stirred for 7 h at 25°C and then filtered. The filtrate was concentrated to dryness under reduced pressure followed by coevaporation with methanol (2x200 mL). The residue was dissolved in methanol (350 mL). The solution was adsorbed on silica gel (175 g) and evaporated to dryness. The mixture was slurried in dichloromethane (500 mL) and applied on top of a silica gel column (1 kg). The column was eluted with a gradient of dichloromethane-methanol (10:1 → 2:1). The less polar 2',3'-dimethyl side product was removed and the co-eluting 2' and 3'-*O*-methyl product containing fractions were combined and evaporated under reduced pressure to a syrup. The syrup was dissolved in a minimum of hot ethanol (ca. 150 mL) and allowed to cool to 25°C. The resulting precipitate (2' less soluble) was collected, washed with ethanol (2x25 ml) and dried to give 15.2 g of pure 2'-*O*-methylcytidine; mp 252-254°C mp 252-254°C); TLC homogenous (Rf 0.50, dichloromethane-methanol 3:1, (Rf of 3' isomer 0.50 and the dimethyl product 0.80). The filtrate was evaporated to give 18 g of a mixture of isomers and sodium iodide.

### Step 2. N4-Benzoyl-2'-O-methylcytidine

The pure 2'-*O*-methylcytidine (15.2 g, 0.060 mol) was dissolved in a solution of benzoic anhydride (14.7 g, 0.12 mol) in dimethylformamide (200 mL) . The solution was stirred at 25°C for 48 h and then evaporated to dryness under reduced pressure. The residue was triturated with methanol (2x200 mL), collected and then triturated with warm ether (300 mL) for 10 min. The solid was collected and triturated with hot 2-propanol (50 mL) and allowed to stand at 4°C for 16 h. The solid was collected and dried to give 17 g of product. The crude filtrate residue (18 g) of 2'-O-methylcytidine was treated with benzoic anhydride (17.3 g, 0.076 mol) in dimethylformamide (250 mL) as above and triturated in a similar fashion to give an additional 6.7 g of pure product for a total yield of 23.7 g (16% from cytidine) of solid; TLC homogenous (Rf 0.25, chloroform-methanol 5:1, co-spots with material made utilizing Method A)

### EXAMPLE 72 (Comparative example)

### N4-Benzoyl-5'-O-(4,4'-dimethoxytriphenylmethyl)-2'-O-methylcytidine

N4-Benzoyl-2'-*O*-methylcytidine, (28 g, 0.077 mol) was evaporated under reduced pressure to an oil with pyridine (400 mL). To the residue was added 4,4'-dimethoxytriphenylmethyl chloride (DMT-Cl, 28.8 g, 0.085 mol) and pyridine (400 mL). The mixture was stirred at 25°C for 2 h and then quenched by the addition of methanol (10 mL) for 30 min. The mixture was concentrated under reduced pressure and the residue was chromatographed on silica gel (500 g, hexanes-ethyl acetate-triethylamine 60:40:1 and then ethyl acetate-triethylamine 99:1). The appropriate fractions were combined, evaporated under reduced pressure and dried at 40°C/0.2 mmHg for 2 h to give 26 g (74%) of foam; TLC homogenous (Rf 0.45, ethyl acetate) ; PMR (DMSO) d 11.3 (H-N⁴), 8.4-6.9 (H-6, H-5, Bz), 5.95 (H-1'), 5.2 (HO-3'), 3.7 (s, 6, CH₃O-trit.), 3 .5 (s, 3, CH₃O-2')

### EXAMPLE 73 (Comparative example)

### N4-Benzoyl-5'-O-(4,4'-dimethoxytriphenylmethyl)-2'-O-methyl cytidine-3'-O-(β-cyanoethyl N,N-diisopropylphosphoramidite)

The product was prepared as per the procedure of Example 38 starting with intermediate compound N4-benzoyl-5'-*O*-(4,4'-dimethoxytriphenylmethyl)-2'-*O*-methylcytidine (22.0 g, 0.0333 mole) and using ethyl acetate-hexanes-triethylamine (59:40:1) as the chromatography eluent to give the product as a solid foam ( 23.6 g) in 83% yield; TLC homogenous diastereomers (Rf 0.46; 0.33, ethyl acetate-hexanes-triethylamine 59:40:1); ³¹P-NMR (CD₃CN, H₃PO₄ std.) d 150.34; 151.02 (diastereomers).

### EXAMPLE 74 (Comparative example)

### 2'-O-Nonylcytidine

Cytidine (10.1 g, 0.0415 mol), sodium hydride (2.0 g, 1.2 eq), iodononane (9.8 ml, 1.2 eq.) in DMF (100 ml) were reacted as per the procedure of Example 71, Step 1 to yield the 2' and 3' isomers as a sticky foam (11.6 g).

### EXAMPLE 75 (Comparative example)

### N4-Benzoyl-2'-O-nonylcytidine

The mixture of 2'-*O*-nonylcytidine and 3'-*O*-nonylcytidine (11.5 g) is converted to N4-benzoyl-2'-*O*-nonylcytidine as per the procedure of Example 71, Step 2.

### EXAMPLE 76 (Comparative example)

### N4-Benzoyl-5'-O-(dimethoxytrityl)-2'-O-nonylcytidine

N4-Benzoyl-2'-*O*-nonylcytidine (2. 67 g, 0. 0056 mol) was treated with dimethoxytrityl chloride (2.0 g, 1.1 eq) as per the procedure of Example 72 to give 4.2 g of pure product. Anal. Calcd. for C₄₆H₅₃N₃O₈·½H₂O: C, 70.39; H, 6.93; N, 5.35. Found: C, 71.20; H, 6.88; N, 5.41.

### EXAMPLE 77 (Comparative example)

### N4-Benzoyl-5'-O-(dimethoxytrityl)-2'-O-nonylcytidine-3'-O-(β-cyanoethyl N,N-diisopropylphosphoramidite)

The product was prepared as per the procedure of Example 38 starting with intermediate compound N4-benzoyl-5'-*O*-(4,4'-dimethoxytriphenylmethyl)-2'-*O*-nonylcytidine (4.1 g, 0.0053 mole) treated with bis-N,N-diisopropylaminocyanoethylphosphite (3.3 ml) and N,N-diisopropylaminohydrotetrazolide (450 mg). The product was eluted from the silica gel column using Hexane/EtOAc (3:1 → 1:1 with 1% triethylamine) as the chromatography eluent to give the product as a solid foam (4.21 g). ³¹P-NMR (CD₃CN, H₃PO₄ std.) shows the diastereomers;

### EXAMPLE 78 (Comparative example)

### 2'-O-Pentylcytidine

Cytidine (10 g, 0.041 mol), sodium hydride (2.4 g, 1.5 eq), bromopentane (7.6 ml, 1.5 eq.) in DMSO (90 ml) were reacted as per the procedure of Example 71, Step 1 to yield the 2' and 3' isomers as a foam (7.6 g).

### EXAMPLE 79 (Comparative example)

### N4-Benzoyl-2'-O-pentylcytidine

The mixture of 2'-*O*-pentylcytidine and 3'-*O*-pentylcytidine (7.5 g) is converted to N4-benzoyl-2'-*O*-pentylcytidine as per the procedure of Example 71, Step 2.

### EXAMPLE 80 (Comparative example)

### N4-Benzoyl-5'-O-(dimethoxytrityl)-2'-O-pentylcytidine

N4-Benzoyl-2'-*O*-pentylcytidine (3.0 g, 0.007 mol) was treated with dimethoxytrityl chloride (2.7 g, 1.1 eq) as per the procedure of Example 72 to give 3.5 g of pure product. Anal. Calcd. for C₄₂H₄₅N₃O₈·½H₂O: C, 69.21; H, 6.36; N, 5.76. Found: C, 69.51; H, 6.30; N, 5.71.

### EXAMPLE 81 (Comparative example)

### N4-Benzoyl-5'-O-(dimethoxytrityl)-2'-O-pentylcytidine-3'-O-(β-cyanoethyl N,N-diisopropylphosphoramidite)

The product was prepared as per the procedure of Example 38 starting with intermediate compound N4-benzoyl-5'-*O*-(4,4'-dimethoxytriphenylmethyl)-2'-*O*-pentylcytidine (3.5 g, 0.0048 mole) treated with bis-N,N-diisopropylaminocyanoethylphosphite (2.9 g, 3.1 ml, 2 eq.) and N,N-diisopropylaminohydrotetrazolide (400 mg, 0.5 eq.). The product was eluted from the silica gel column using Hexane/EtOAc (3:1 → 1:1 with 1% triethylamine) as the chromatography eluent to give the product as a solid foam (3.24 g). ³¹P-NMR (CD₃CN, H₃PO₄ std.) shows the diastereomers.

### EXAMPLE 82 (Comparative example)

### 2'-O-Propylcytidine

Cytidine (16.5 g, 0.068 mol) was treated with sodium hydride (4.5 g) and bromopropane (15 ml) in DMF (150 ml) at room temperature for three days. The resulting reaction mixture was used directly in the next step (see Example 83).

### EXAMPLE 83 (Comparative example)

### N4-Benzoyl-2'-O-propylcytidine

To the 2'-*O*-propylcytidine reaction mixture of Example 82 in an ice bath was added pyridine (60 ml) and trimethylsilyl chloride (60 ml). The reaction was stirred for 30 mins followed by the addition of benzoyl chloride (55 ml). The resulting reaction mixture was stirred for 2.5 hrs and then cooled in an ice bath. H₂O (100 ml) and cone. NH₄OH (100 ml) were added. After stirring for 30 mins, the reaction mixture was evaporated and the residue partition between H₂O and CH₂Cl₂. The organic phase was washed once with dil Na₂CO₃, once with dil HCl, dried over MgSO₄ and evaporated. The resulting residue was loaded on a silica gel column (150 g) and eluted with first CH₂Cl₂ then 5 to 10% MeOH in CH₂Cl₂ as the elution solvent. The product containing fractions were evaporated to a foam. The foam was crystallized from EtOAc/Hexanes to give the product (6.5 g total) in several crystal batches.

### EXAMPLE 84 (Comparative example)

### N4-Benzoyl-5'-O-(dimethoxytrityl)-2'-O-propylcytidine

N4-Benzoyl-2'-*O*-propylcytidine (3.0 g, 0.007 mol) was treated with dimethoxytrityl chloride (1.5 g) as per the procedure of Example 72 to give 1.5 g of pure product. Anal. Calcd. for C₄₀H₄₂N₃O₈·½H₂O: C, 68.45; H, 6.18; N, 5.99. Found: C, 68.39; H, 5.99; N, 5.95.

### EXAMPLE 85 (Comparative example)

### N4-Benzoyl-5'-O-(dimethoxytrityl)-2'-O-propylcytidine-3'-O-(β-cyanoethyl N,N-diisopropylphosphoramidite)

The product was prepared as per the procedure of Example 38 starting with intermediate compound N4-benzoyl-5'-*O*-(4,4'-dimethoxytriphenylmethyl)-2'-*O*-propylcytidine (3.8 g, 0.0055 mole) treated with bis-N,N-diisopropylaminocyanoethylphosphite (3.5 ml, 2 eq.) and N,N-diisopropylaminohydrotetrazolide (500 mg, 0.5 eq.). The product was eluted from the silica gel column using Hexane/EtOAc (1:1 with 1% triethylamine) as the chromatography eluent to give the product as a solid foam (4.72 g). ³¹P-NMR (CD₃CN, H₃PO₄ std.) shows the diastereomers.

### EXAMPLE 86

### N2,N6-Diisobutyrylamino-9-(2'-O-methyl-β-D-ribofuranosyl)purine

N2,N6-Diamino-9-(2'-*O*-methyl-β-*D*-ribofuranosyl)purine (1.6 g, 5.39 mmol, see Example 34) was co-evaporated with pyridine (25 ml). A suspension of the residue in pyridine (40 ml) was cooled in an ice bath and trimethylsilyl chloride (4.8 ml) was added. The reaction mixture was stirred for 30 mins followed by the addition of butyryl chloride (2.8 ml, 5 eq). The resulting reaction mixture was stirred at room temperature for 4 hours. H₂O (10 ml) and conc. NH₄OH (10 ml) were added with stirring to quench the reaction mixture. After 30 mins, the reaction mixture was evaporated and the residue purified on a silica gel column using CH₂Cl₂ → 10% MeOH/CH₂Cl₂ to elute the product. The appropriate fractions were evaporated to yield the product as an oil (2.4 g).

### EXAMPLE 87

### N2,N6-Diisobutyrylamino-9-(5'-O-dimethoxytrityl-2'-O-methyl-β-D-ribofuranosyl)purine

N2,N6-Diisobutyrylamino-9-(2'-*O*-methyl-β-*D*-ribofuranosyl)purine (2.4 g) was co-evaporated with pyridine and redissolved in pyridine. Dimethoxytrityl chloride (1.8 g, 1 eq) and dimethylaminopyridine (5 mg) were added and the resulting solution was stirred overnight at room temperature. The solvent was partly evaporated and the residue partition between CH₂Cl₂ - dil. Na₂CO₃. The organic phase was washed with dil. Na₂CO₃, dried with MgSO₄ and evaporated. The residue was purified on a silica gel column eluted with Hexanes/EtOAc (1:1) containing 1% triethylamine. The fraction contain the product were evaporated to yield the product as a foam (2.4 g).

### EXAMPLE 88

### N2,N6-Diisobutyrylamino-9-[5'-O-dimethoxytrityl-2'-O-methyl-3'-O-(β-cyanoethyl N,N-diisopropylphosphoramide)-β-D-ribofuranosyl]purine

N2,N6-Diisobutyrylamino-9-(5'-*O*-dimethoxytrityl-2'-*O*-methyl-β-*D*-ribofuranosyl)purine (1.7 g, 0.0023 mol) was treated with bis-N,N-diisopropylaminocyanoethylphosphite (1.48 ml, 2 eq.) and N,N-diisopropylaminohydrotetrazolide (200 mg) at room temperature overnight. The reaction mixture was partitioned between dil. Na₂CO₃/CH₂Cl₂, the organic phase was dried over MgSO₄ and evaporated. The residue was loaded on a silica gel column and eluted with Hexanes/EtOAc (3:1 → 1:1 with 1% triethylamine) to give the product as a solid foam (1.73 g). ³¹P-NMR (CD₃CN, H₃PO₄ std.) shows the diastereomers.

### EXAMPLE 89

### Oligonucleotide Synthesis

Once nucleoside phosphoramidites of the invention have been prepared, they can then subsequently be incorporated into oligonucleotides, which are synthesized by a standard solid phase, automated nucleic acid synthesizer such as the Applied Biosystems, Incorporated 380B or MilliGen/Biosearch 7500 or 8800. Standard phosphoramidite coupling chemistries (*see, e.g*., M. Caruthers, *Oligonucleotides. Antisense Inhibitors of Gene Expression*., pp. 7-24, J.S. Cohen, ed. , CRC Press, Inc. Boca Raton, FL, 1989) are used with these synthesizers to provide the desired oligonucleotides. The Beaucage reagent (*see, e.g., J. Am. Chem. Soc*. **1990**, *112*, 1253) or elemental sulfur (*see, e.g., Tetrahedron Letters* **1981**, *22*, 1859), can likewise be used to provide phosphorothioate oligonucleotides.

### EXAMPLE 90

### A. Evaluation of the thermodynamics of hybridization of 2'- modified oligonucleotides.

The ability of the 2'- modified oligonucleotides to hybridize to their complementary RNA or DNA sequences is determined by thermal melting analysis. The RNA complement is synthesized from T7 RNA polymerase and a template-promoter of DNA synthesized with an Applied Biosystems, Inc. 380B RNA species was purified by ion exchange using FPLC (LKB Pharmacia, Inc.). Natural antisense oligonucleotides or those containing 2'-O-alkyl guanosine at specific locations are added to either the RNA or DNA complement at stoichiometric concentrations and the absorbance (260 nm) hyperchromicity upon duplex to random coil transition was monitored using a Gilford Response II spectrophotometer. These measurements are performed in a buffer of 10 mM Na-phosphate, pH 7.4, 0.1 mM EDTA, and NaCl to yield an ionic strength of 10 either 0.1 M or 1.0 M. Data is analyzed by a graphic representation of 1/Tₘ vs ln[Ct], where [Ct] was the total oligonucleotide concentration. From this analysis the thermodynamic para-meters is determined. Based upon the information gained concerning the stability of the duplex of heteroduplex formed, the placement of 2'-O-alkyl guanosine into oligonucleotides are assessed for their effects on helix stability. Modifications that drastically alter the stability of the hybrid exhibit reductions in the free energy (delta G) and decisions concerning their usefulness as antisense oligonucleotides are made.

### B. Fidelity of hybridization of 2'- modified oligonucleotides

The ability of the 2'-O-alkyl guanosine modified antisense oligo- nucleotides to hybridize with absolute specificity to the targeted mRNA is shown by Northern blot analysis of purified target mRNA in the presence of total cellular RNA. Target mRNA is synthesized from a vector containing the cDNA for the target mRNA located downstream from a T7 RNA polymerase promoter. Synthesized mRNA was electrophoresed in an agarose gel and transferred to a suitable support membrane (ie. nitrocellulose). The support membrane was blocked and probed using [³²P]-labeled antisense oligonucleotides. The stringency will be determined by replicate blots and washing in either elevated temperatures or decreased ionic strength of the wash buffer. Autoradiography was performed to assess the presence of heteroduplex formation and the autoradiogram quantitated by laser densitometry (LKB Pharmacia, Inc.). The specificity of hybrid formation was determined by isolation of total cellular RNA by standard techniques and its analysis by agarose electrophoresis, membrane transfer and probing with the labeled 2'-modified oligonucleotides. Stringency was predetermined for the unmodified antisense oligonucleotides and the conditions used such that only the specifically targeted mRNA was capable of forming a heteroduplex with the 2'-modified oligonucleotide.

### EXAMPLE 91 - Nuclease Resistance

### A. Evaluation of the resistance of 2'- modified oligonucleotides to serum and cytoplasmic nucleases.

Natural phosphorothioate, and 2- modified oligonucleotides were assessed for their resistance to serum nucleases by incubation of the oligonucleotides in media containing various concentrations of fetal calf serum or adult human serum. Labeled oligonucleotides were incubated for various times, treated with protease K and then analyzed by gel electrophoresis on 20% polyacrylamine-urea denaturing gels and subsequent autoradiography. Autoradiograms were quantitated by laser densitometry. Based upon the location of the modifications and the known length of the oligonucleotide it was possible to determine the effect on nuclease degradation by the particular 2'-modification. For the cytoplasmic nucleases, a HL60 cell line was used. A post-mitochondrial supernatant was prepared by differential centrifugation and the labeled oligonucleotides were incubated in this supernatant for various times. Following the incubation, oligo-nucleotides were assessed for degradation as outlined above for serum nucleolytic degradation. Autoradiography results were quantitated for comparison of the unmodified, the phosphorothioates, and the 2'- modified oligonucleotides.

### B. Evaluation of the resistance of 2'- modified oligonucleotides to specific endo- and exo-nucleases.

Evaluation of the resistance of natural and 2'-modified oligonucleotides to specific nucleases (ie, endonucleases, 3',5'-exo-, and 5',3'-exonucleases) was done to determine the exact effect of the modifications on degradation. Modified oligonucleotides were incubated in defined reaction buffers specific for various selected nucleases. Following treatment of the products with proteinase K, urea was added and analysis on 20% poly- acrylamide gels containing urea was done. Gel products were visualized by staining using Stains All (Sigma Chemical Co.) . Laser densitometry was used to quantitate the extend of degradation. The effects of the 2'-modifications were determined for specific nucleases and compared with the results obtained from the serum and cytoplasmic systems.

## Claims

1. A process for preparing a 2'-*O*-alkylated guanosine 3'-*O*-phosphoramidite comprising the steps of:
alkylating a 2,6-diamino-9-(ribofuranosyl)purine with an alkylating agent X-L, wherein X is R₁-(R₂)ₙ; R₁ is C₃-C₂₀ alkyl, C₄-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to about 6; and L is a leaving group; in the presence of a metal hydride to form a 2,6-diamino-9-(2'-*O*-alkylated ribofuranosyl)purine;
deaminating said 2,6-diamino-9-(2'-*O*-alkylated ribofuranosyl)purine to form a 2'-*O*-alkylated guanosine;
blocking the 5'-hydroxyl moiety of said 2'-*O*-alkylated guanosine; and
phosphitylating the 3'-position of said 5'-blocked 2'-*O*-alkylated guanosine.

2. The process of claim 1, wherein said blocking step adds a dimethoxytrityl group.

3. The process of claim 1, wherein said phosphitylation step is conducted with bis-N,N-diisopropylaminocyanoethylphosphite.

4. The process of claim 3, wherein said phosphitylation step is conducted in the presence of N,N-diisopropylaminohydrotetrazolide.

5. The process of claim 1, further comprising blocking the N2-amino moiety of said 2'-*O*-alkylated guanosine prior to blocking said 5'-hydroxyl moiety.

6. The process of claim 1, wherein said deamination step is performed using adenosine deaminase.

7. A process for preparing 2'-*O*-substituted guanosine comprising:
treating 2,6-diaminopurine riboside with a base having a pK_{b} value greater than or equal to the pK_{b} value of NaH and with an alkylating agent X-L, wherein X is R₁-(R₂)ₙ; R₁ is C₃-C₂₀ alkyl, C₄-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to about 6; and L is a leaving group; to form 2'-*O*-substituted-2,6-diaminopurine riboside and 3'-*O*-substituted-2,6-diaminopurine riboside wherein said substituent is said group X;
deaminating said 2'-*O*-substituted-2,6-diaminopurine riboside to yield the said 2'-*O*-substituted guanosine; and
recovering said 2'-*O*-substituted guanosine.

8. The process of claim 7, further comprising:
separating said 2'-*O*-substituted-2,6-diaminopurine riboside from said 3'-*O*-substituted-2,6-diaminopurine riboside.

9. The process of claim 8, wherein said 2'-*O*-substituted-2,6-diaminopurine riboside is separated from said 3'-*O*-substituted-2,6-diaminopurine riboside by conducting said deamination under conditions such that the rate of deamination of said 2'-*O*-substituted-2,6-diaminopurine riboside is accelerated compared to the rate of deamination of said 3'-*O*-substituted-2,6-diaminopurine riboside.

10. The process of claim 7, wherein said 2'-*O*-substituted-2,6-diaminopurine is deaminated with adenosine deaminase.

11. The process of claim 9, wherein said accelerated rate of deamination of said 2'-*O*-substituted-2,6-diaminopurine riboside is achieved by use of the enzyme adenosine deaminase.

12. The process of claim 7, wherein said base is sodium hydride.

13. The process of claim 7, wherein said 2'-*O*-substituted-2,6-diaminopurine riboside is physically separated from said 3'-*O*-substituted-2,6-diaminopurine riboside by crystallization or chromatography.

14. A process for preparing 2'-*O*-alkyl-guanosine comprising:
treating 2,6-diaminopurine riboside with a base having a pK_{b} value greater than or equal to the pK_{b} value of NaH and with an alkylating agent X-L, wherein X is R₁-(R₂)ₙ; R₁ is C₃-C₂₀ alkyl, C₄-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to about 6; and L is a leaving group; to form 2'-*O*-alkyl-2,6-diaminopurine riboside;
treating said 2'-*O*-alkyl-2,6-diaminopurine riboside with adenosine deaminase; and
recovering said 2'-*O*-alkyl guanosine.

15. The process of claim 14, wherein said base is sodium hydride.

16. The process of claim 14, further comprising:
separating said 2'-*O*-alkyl-2,6-diaminopurine riboside from further alkylated products resulting from said treatment of said 2,6,-diaminopurine riboside with said base and said alkylating agent X-L.

17. A process for preparing a 3'-*O*-phosphoramidite of 2'-*O*-alkyl guanosine comprising:
treating 2,6-diaminopurine riboside with a base having a pK_{b} value greater than or equal to the pK_{b} value of NaH and with an alkylating agent X-L, wherein X is R₁-(R₂)ₙ; R₁ is C₃-C₂₀ alkyl, C₄-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to about 6; and L is a leaving group; to form 2'-*O*-alkyl-2,6-diaminopurine riboside;
treating said 2'-*O*-alkyl-2,6-diaminopurine riboside with adenosine deaminase to form 2'-*O*-alkyl-guanosine;
protecting the 2-NH₂ moiety of said 2'-*O*-alkyl-guanosine with a protecting group;
protecting the 5'-OH moiety of said 2'-*O*-alkyl-guanosine with a further protecting group;
phosphitylating the 3'-OH moiety of said protected 2'-*O*-alkyl-guanosine; and
recovering said 3'-*O*-phosphoramidite of 2'-*O*-alkyl guanosine.

18. The process of claim 17, wherein said protected 2'-*O*-alkyl guanosine is phosphitylated with 2-cyanoethyl N,N-diisopropylaminochlorophosphine.

19. The process of claim 17, wherein said base is sodium hydride.

20. A process for preparing an oligonucleotide that comprises at least one 2'-*O*-alkylated guanosine nucleotide comprising:
alkylating a 2,6-diamino-9-(ribofuranosyl)purine with a base having a pK_{b} value greater than or equal to the pK_{b} value of NaH and with an alkylating agent X-L, wherein X is R₁-(R₂)ₙ; R₁ is C₃-C₂₀ alkyl, C₄-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to about 6; and L is a leaving group; to form a 2,6-diamino-9-(2'-*O*-alkylated ribofuranosyl)purine;
deaminating said 2,6-diamino-9-(2'-*O*-alkylated ribofuranosyl)purine to form a 2'-*O*-alkylated guanosine;
blocking the 5'-hydroxyl moiety of said 2'-*O*-alkylated guanosine;
phosphitylating the 3'-position of said 5'-blocked 2'-*O*-alkylated guanosine to form a 2'-*O*-alkylated guanosine 3'-*O*-phosphoramidite; and
coupling said 2'-*O*-alkylated guanosine 3'-*O*-phosphoramidite with a 5'-hydroxyl moiety on an oligonucleotide utilizing phosphoramidite coupling conditions.

## Patentansprüche

1. Verfahren zur Herstellung eines 2'-O-alkylierten Guanosin 3'-Ophosphoramidits, umfassend die Schritte:
des Alkylierens eines 2,6-Diamino-9-(ribofuranosyl)purins mit einem Alkylierungsmittel X-L, wobei X R₁-(R₂)ₙ ist, R₁ eine C₃-C₂₀-Alkyl-, eine C₄-C₂₀-Alkenyl- oder eine C₂-C₂₀-Alkinylgruppe ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Intercalator, Reportermolekül, Konjugat, Polyamin, Polyamid, Polyalkylenglykol oder Polyether ist, n eine ganze Zahl von 0 bis etwa 6 ist, und L eine Abgangsgruppe ist, in der Gegenwart eines Metallhydrids, um ein 2,6-Diamino-9-(2'-O-alkyliertes Ribofuranosyl)purin zu bilden,
des Desaminierens des 2,6-Diamino-9-(2'-O-alkylierten Ribofuranosyl)purins, um ein 2'-O-alkyliertes Guanosin zu bilden,
des Blockierens der 5'-Hydroxylgruppe des 2'-O-alkylierten Guanosins, und
des Phosphitylierens der 3'-Position des 5'-blockierten 2'-O-alkylierten Guanosins.

2. Verfahren nach Anspruch 1, wobei der Blockierungsschritt eine Dimethoxytritylgruppe hinzufügt.

3. Verfahren nach Anspruch 1, wobei der Phosphitylierungsschritt mit Bis-N,N-diisopropylaminocyanoethylphosphit durchgeführt wird.

4. Verfahren nach Anspruch 3, wobei der Phosphitylierungsschritt in Gegenwart von N,N-Diisopropylaminohydrotetrazolid durchgeführt wird.

5. Verfahren nach Anspruch 1, ferner umfassend das Blockieren der N2-Aminogruppe des 2'-O-alkylierten Guanosins vor dem Blockieren der 5'-Hydroxylgruppe.

6. Verfahren nach Anspruch 1, wobei der Desaminierungsschritt unter Verwendung von Adenosindesaminase durchgeführt wird.

7. Verfahren zur Herstellung eines 2'-O-substituierten Guanosins, umfassend:
das Behandeln von 2,6-Diaminopurinribosid mit einer Base mit einem pK_{b}-Wert von größer oder gleich dem pK_{b}-Wert von NaH und mit einem Alkylierungsmittel X-L, wobei X R₁-(R₂)ₙ ist, R₁ eine C₃-C₂₀-Alkyl-, eine C₄-C₂₀-Alkenyl- oder C₂-C₂₀-Alkinylgruppe ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Intercalator, Reportermolekül, Konjugat, Polyamin, Polyamid, Polyalkylenglykol oder Polyether ist, wobei n eine ganze Zahl von 0 bis etwa 6 ist, und L eine Abgangsgruppe ist, um 2'-O-substituiertes 2,6-Diaminopurinribosid und 3'-O-substituiertes 2,6-Diaminopurinribosid zu bilden, wobei der Substituent die Gruppe X ist,
des Desaminierens des 2'-O-substituierten 2,6-Diaminopurinribosids, um das 2'-O-substituierte Guanosin zu ergeben, und
das Gewinnen des 2'-O-substituierten Guanosins.

8. Verfahren nach Anspruch 7, ferner umfassend:
das Trennen des 2'-O-substituierten 2,6-Diaminopurinribosids von dem 3'-O-substituierten 2,6-Diaminopurinribosid.

9. Verfahren nach Anspruch 8, wobei das 2'-O-substituierte 2,6-Diaminopurinribosid von dem 3'-O-substituierten 2,6-Diaminopurinribosid durch Durchführen der Desaminierung unter Bedingungen derart getrennt wird, daß die Desaminierungsrate des 2'-O-substituierten 2,6-Diaminopurinribosids verglichen mit der Desaminierungsrate des 3'-O-substituierten 2,6-Diaminopurinribosids beschleunigt ist.

10. Verfahren nach Anspruch 7, wobei das 2'-O-substituierte 2,6-Diaminopurin mit Adenosindesaminase desaminiert wird.

11. Verfahren nach Anspruch 9, wobei die beschleunigte Desaminierungsrate des 2'-O-substituierten 2,6-Diaminopurinribosids durch Verwendung des Enzyms Adenosindesaminase erreicht wird.

12. Verfahren nach Anspruch 7, wobei die Base Natriumhydrid ist.

13. Verfahren nach Anspruch 7, wobei das 2'-O-substituierte 2,6-Diaminopurinribosid von dem 3'-O-substituierten 2,6-Diaminopurinribosid durch Kristallisation oder Chromatographie physikalisch getrennt wird.

14. Verfahren zur Herstellung von 2'-O-Alkyl-Guanosin, umfassend:
das Behandeln von 2,6-Diaminopurinribosid mit einer Base mit einem pK_{b}-Wert von größer oder gleich dem pK_{b}-Wert von NaH und mit einem Alkylierungsmittel X-L, wobei X R₁-(R₂)ₙ ist, R₁ eine C₃-C₂₀-Alkyl-, C₄-C₂₀-Alkenyloder C₂-C₂₀-Alkinylgruppe ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Intercalator, Reportermolekül, Konjugat, Polyamin, Polyamid, Polyalkylenglykol oder Polyether ist, wobei n eine ganze Zahl von 0 bis etwa 6 ist, und L eine Abgangsgruppe ist, um 2'-O-Alkyl-2,6-diaminopurinribosid zu bilden,
das Behandeln des 2'-O-Alkyl-2,6-diaminopurinribosids mit Adenosindesaminase, und
das Gewinnen des 2'-O-Alkylguanosins.

15. Verfahren nach Anspruch 14, wobei die Base Natriumhydrid ist.

16. Verfahren nach Anspruch 14, ferner umfassend:
das Trennen des 2'-O-Alkyl-2,6-diaminopurinribosids von weiteren alkylierten Produkten, die von der Behandlung des 2,6-Diaminopurinribosids mit der Base und dem Alkylierungsmittel X-L resultieren.

17. Verfahren zur Herstellung eines 3'-O-Phosphoramidits von 2'-O-Alkylguanosin, umfassend:
das Behandeln von 2,6-Diaminopurinribosid mit einer Base mit einem pK_{b}-Wert von größer oder gleich dem pK_{b}-Wert von NaH und mit einem Alkylierungsmittel X-L, wobei X R₁-(R₂)ₙ ist, R₁ eine C₃-C₂₀-Alkyl-, eine C₄-C₂₀-Alkenyl- oder eine C₂-C₂₀-Alkinylgruppe ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Intercalator, Reportermolekül, Konjugat, Polyamin, Polyamid, Polyalkylenglykol oder Polyether ist, wobei n eine ganze Zahl von 0 bis etwa 6 ist, und L eine Abgangsgruppe ist, um 2'-O-Alkyl-2,6-diaminopurinribosid zu bilden,
das Behandeln des 2'-O-Alkyl-2,6-diaminopurinribosids mit Adenosindesaminase, um 2'-O-Alkylguanosin zu bilden,
das Schützen der 2-NH₂-Gruppe des 2'-O-Alkylguanosins mit einer Schutzgruppe,
das Schützen der 5'-OH-Gruppe des 2'-O-Alkylguanosins mit einer weiteren Schutzgruppe,
das Phosphitylieren der 3'-OH-Gruppe des geschützten 2'-O-Alkylguanosins, und
das Gewinnen des 3'-O-Phosphoramidits des 2'-O-Alkylguanosins.

18. Verfahren nach Anspruch 17, wobei das geschützte 2'-O-Alkylguanosin mit 2-Cyanoethyl-N,N-diisopropylaminochlorphosphin phosphityliert wird.

19. Verfahren nach Anspruch 17, wobei die Base Natriumhydrid ist.

20. Verfahren zur Herstellung eines Oligonucleotids, das mindestens ein 2'-O-alkyliertes Guanosinnucleotid umfaßt, umfassend:
das Alkylieren eines 2,6-Diamino-9-(ribofuranosyl)purins mit einer Base mit einem pK_{b}-Wert von größer oder gleich dem pK_{b}-Wert von NaH und mit einem Alkylierungsmittel X-L, wobei X R₁-(R₂)ₙ ist, R₁ eine C₃-C₂₀-Alkyl-, eine C₄-C₂₀-Alkenyl- oder eine C₂-C₂₀-Alkinylgruppe ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Intercalator, Reportermolekül, Konjugat, Polyamin, Polyamid, Polyalkylenglykol oder Polyether ist, wobei n eine ganze Zahl von 0 bis etwa 6 ist, und L eine Abgangsgruppe ist, um ein 2,6-Diamino-9-(2'-Oalkyliertes Ribofuranosyl)purin zu bilden,
das Desaminieren des 2,6-Diamino-9-(2'-O-alkylierten Ribofuranosyl)purins, um ein 2'-O-alkyliertes Guanosin zu bilden,
das Blockieren der 5'-Hydroxylgruppe des 2'-O-alkylierten Guanosins,
das Phosphitylieren der 3'-Position des 5'-blockierten 2'-O-alkylierten Guanosins, um ein 2'-O-alkyliertes Guanosin 3'-O-Phosphoramidit zu bilden, und
das Kuppeln des 2'-O-alkylierten Guanosin 3'-O-Phosphoramidits mit einer 5'-Hydroxylgruppe an ein Oligonucleotid unter Verwendung von Phosphoramidit-Kupplungsbedingungen.

## Revendications

1. Procédé de préparation d'une guanosine 2'-*O*-alkylée 3'-*O*-phosphoramidite comprenant les étapes de :
■ alkylation d'une 2,6-diamino-9-(ribofuranosyl)purine avec un agent alkylant X-L, dans lequel X est R₁-(R₂)ₙ; R₁ est un groupe alkyle en C₃-C₂₀, alkényle en C₄-C₂₀ ou alkynyle en C₂-C₂₀ ; R₂ est un groupe halogène, hydroxyle, thiol, cétone, carboxyle, nitro, nitrosé, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phthalimido, imidazole, azido, hydrazino, hydroxylamino, isocyanato, sulfoxide, sulfone, sulfide, disulfide, silyle, aryle, hétérocycle, carbocycle, intercalateur, molécule rapporteuse, conjugué, polyamine, polyamide, polyalkylène glycol, ou polyester ; n est un nombre entier compris entre 0 et environ 6 ; et L est un groupe partant ; en présence d'un métal hybride pour former une 2,6-diamino-9-(2'-*O*-alkylée ribofuranosyl)purine ;
■ désamination de ladite 2,6-diamino-9-(2'-*O*-alkylée ribofuranosyl)purine pour former une guanosine 2'-*O*-alkylée;
■ blocage du groupement 5'-hydroxyl de ladite guanosine 2'-*O*-alkylée ; et
■ phosphatisation de la 3'-position de ladite guanosine 5'-bloquée 2'-*O*-alkylée.

2. Procédé selon la revendication 1, dans lequel ladite étape de blocage ajoute un groupe diméthoxytrityl.

3. Procédé selon la revendication 1, dans lequel ladite étape de phosphatisation est conduite avec un bis-N,N-diisopropylaminocyanocthylphosphite.

4. Procédé selon la revendication 3, dans lequel ladite étape de phosphatisation est conduite en présence de N,N-diisopropylaminohydrotétrazolide.

5. Procédé selon la revendication 1, comprenant également le blocage dudit groupement N2-amino de ladite guanosine 2'-*O*-alkylée avant le blocage dudit groupement 5'-hydroxyl.

6. Procédé selon la revendication 1, dans lequel ladite étape de désamination est réalisée en utilisant de l'adénosine désaminase.

7. Procédé de préparation d'une guanosine 2'-*O*-substituée comprenant :
■ le traitement d'un riboside 2,6-diaminopurine avec une base ayant une valeur pKₙ supérieure ou égale à la valeur pK_{b} de NaH et avec un agent alkylant X-L, dans lequel X est R₁-(R₂)ₙ ; R₁ est un groupe alkyle en C₃-C₂₀, alkényle en C₄-C₂₀ ou alkynyle en C₂-C₂₀ ; R₂ est un groupe halogène, hydroxyle, thiol, cétone, carboxyle, nitro, nitrosé, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxide, sulfone, sulfide, disulfide, silyle, aryle, hétérocycle, carbocycle, intercalateur, molécule rapporteuse, conjugué, polyamine, polyamide, polyalkylène glycol, ou polyester ; n est un nombre entier compris entre 0 et environ 6 ; et L est un groupe partant ; pour former un riboside 2'-*O*-substitué-2,6-diaminopurine et un riboside 3'-*O*-substitué-2,6-diaminopurine dans lequel ledit substituant est ledit groupe X ;
■ la désamination dudit riboside 2'-*O*-substitué-2,6-diaminopurine pour obtenir ladite guanosine 2'-*O*-subsituée ;
■ la récupération de ladite guanosine 2'-*O*-subsituée.

8. Procédé selon la revendication 7, comprenant également :
■ la séparation dudit riboside 2'-*O*-substitué-2,6-diaminopurine d'avec ledit riboside 3'-*O*-substitué-2,6-diaminopurine.

9. Procédé selon la revendication 8, dans lequel ledit riboside 2'-*O*-substituée-2,6-diaminopurine est séparé dudit riboside 3'-*O*-substitué-2,6-diaminopurine en conduisant ladite désamination sous des conditions telles que le taux de désamination dudit riboside 2'-*O*-substitué-2,6-diaminopurine est accéléré en comparaison avec le taux de désamination dudit riboside 3'-*O*-substitué-2,6-diaminopurine.

10. Procédé selon la revendication 7, dans lequel ladite 2'-*O*-substituée-2,6-diaminopurine est désaminée avec de l'adénosine désaminase.

11. Procédé selon la revendication 9, dans lequel le taux de désamination accéléré dudit riboside 2'-*O*-substitué-2,6-diaminopurine est réalisé en utilisant l'enzyme d'adénosine désaminase.

12. Procédé selon la revendication 7, dans lequel ladite base est une hydrure de sodium.

13. Procédé selon la revendication 7, dans lequel ledit riboside 2'-*O*-substitué-2,6-diaminopurine est séparé physiquement dudit riboside 3'-*O*-substitué-2,6-diaminopurine par cristallisation ou chromatographie.

14. Procédé de préparation d'une guanosine 2'-*O*-alkyle comprenant :
■ le traitement d'un riboside 2,6-diaminopurine avec une base ayant une valeur pK_{b} supérieure ou égale à la valeur pK_{b} de NaH et avec un agent alkylant X-L, dans lequel X est R₁-(R₂)ₙ ; R₁ est un groupe alkyle en C₃-C₂₀, alkényle en C₄-C₂₀ ou alkynyle en C₂-C₂₀ ; R₂ est un groupe halogène, hydroxyle, thiol, cétone, carboxyle, nitro, nitrosé, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxide, sulfone, sulfide, disulfide, silyle, aryle, hétérocycle, carbocycle, intercalateur, molécule rapporteuse, conjugué, polyamine, polyamide, polyalkylène glycol, ou polyester ; n est un nombre entier compris entre 0 et environ 6 ; et L est un groupe partant ; pour former un riboside 2'-*O*-alkyle-2,6-diaminopurine ;
■ le traitement dudit riboside 2'-*O*-alkyle-2,6-diaminopurine avec de l'adénosine désaminase ; et
■ la récupération de ladite guanosine 2'-*O*-alkylée.

15. Procédé selon la revendication 14, dans lequel ladite base est une hydrure de sodium.

16. Procédé selon la revendication 14, comprenant également :
■ la séparation dudit riboside 2'-*O*-substitué-2,6-diaminopurine avec d'autres produits alkylés résultant dudit traitement dudit riboside 2,6-diaminopurine avec ladite base et ledit agent alkylant X-L.

17. Procédé de préparation d'une 3'-*O*-phosphoramidite de guanosine 2'-*O*-alkyle comprenant :
■ le traitement d'un riboside 2,6-diaminopurine avec une base ayant une valeur pKₙ supérieure ou égale à la valeur pK_{b} de NaH et avec un agent alkylant X-L, dans lequel X est R₁-(R₂)ₙ ; R₁ est un groupe alkyle en C₃-C₂₀, alkényle en C₄-C₂₀ ou alkynyle en C₂-C₂₀ ; R₂ est un groupe halogène, hydroxyle, thiol, cétone, carboxyle, nitro, nitrosé, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxide, sulfone, sulfide, disulfide, silyle, aryle, hétérocycle, carbocycle, intercalateur, molécule rapporteuse, conjugué, polyamine, polyamide, polyalkylène glycol, ou polyester ; n est un nombre entier compris entre 0 et environ 6 ; et L est un groupe partant ; pour former un riboside 2'-*O*-alkyle-2,6-diaminopurine ;
■ le traitement dudit riboside 2'-*O*-alkyle-2,6-diaminopurine avec de l'adénosine désaminase pour former une guanosine 2'-*O*-aklyle ;
■ la protection du groupement 2-NH₂ de ladite guanosine 2'-*O*-alkyle avec un groupe protecteur ;
■ la protection du groupement 5'-OH de ladite guanosine 2'-*O*-alkyle avec un autre groupe protecteur ;
■ la phosphatisation du groupement 3'-OH de ladite guanosine 2'-*O*-alkyle ; et
■ la récupération de ladite 3'-O-phosphoramidite de guanosine 2'-*O*-alkyle.

18. Procédé selon la revendication 17, dans lequel ladite guanosine 2'-*O*-alkyle est phosphatisée avec 2-cyanoéthyl N,N-diisopropylaminochlorophosphine.

19. Procédé selon la revendication 17, dans lequel ladite base est une hydrure de sodium.

20. Procédé de préparation d'un oligonucléotide qui comprend au moins un nucléotide de guanosine 2'-*O*-alkylée, comprenant :
■ l'alkylation d'un 2,6-diamino-9-(ribofuranosyl)purine avec une base ayant une valeur pK_{b} supérieure ou égale à la valeur pK_{b} de NaH et avec un agent alkylant X-L, dans lequel X est R₁-(R₂)ₙ ; R₁ est un groupe alkyle en C₃-C₂₀, alkényle en C₄-C₂₀ ou alkynyle en C₂-C₂₀ ; R₂ est un groupe halogène, hydroxyle, thiol, cétone, carboxyle, nitro, nitrosé, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxide, sulfone, sulfide, disulfide, silyle, aryle, hétérocycle, carbocycle, intercalateur, molécule rapporteuse, conjugué, polyamine, polyamide, polyalkylène glycol, ou polyester ; n est un nombre entier compris entre 0 et environ 6 ; et L est un groupe partant ; pour former une 2,6-diamino-9-(2'-*O*-alkylée ribofuranosyl)purine ;
■ la désamination de ladite 2,6-diamino-9-(2'-*O*-alkylée ribofuranosyl)purine pour former une guanosine 2'-*O*-alkylée ;
■ le blocage du groupement 5'-hydroxyl de ladite guanosine 2'-*O*-alkylée ;
■ la phosphatisation de la 3'-position de ladite guanosine 5'-bloquée 2'-*O*-alkylée pour former une guanosine 2'-*O*-alkylée 3'-*O*-phosphoramidite ; et
■ le couplage de ladite guanosine 2'-*O*-alkylée 3'-*O*-phosphoramidite avec le groupement 5'-hydroxyl sur un oligonucléotide utilisant des conditions de couplage de la phosphoramidite.
